# EUROPEAN PATENT SPECIFICATION

(11) **EP 2 450 430 B1**
(45) Date of publication and mention of the grant of the patent: **18.03.2015**
(21) Application number: 10794054.6
(22) Date of filing: 24.06.2010
(51) Int. Cl.: C12N 5/00, C12N 5/10, C12Q 1/02, G01N 33/50, G01N 33/68, C12N 15/09

(54) **BIOASSAY METHOD FOR ANTIBODY AGAINST THYROID-STIMULATING HORMONE RECEPTOR, MEASUREMENT KIT FOR THE ANTIBODY, AND NOVEL GENETICALLY MODIFIED CELL FOR USE IN THE BIOASSAY METHOD OR THE MEASUREMENT KIT**
BIOASSAY-VERFAHREN FÜR ANTIKÖRPER GEGEN DEN REZEPTOR DES SCHILDDRÜSENSTIMULIERENDEN HORMONS, MESSKIT FÜR DIE ANTIKÖRPER UND NEUE GENETISCH MODIFIZIERTE ZELLE ZUR VERWENDUNG IN DEM BIOASSAY-VERFAHREN ODER DEM MESSKIT
PROCÉDÉ DE BIODOSAGE D'ANTICORPS ANTI-RÉCEPTEURS DE LA THYRÉOSTIMULINE, NÉCESSAIRE DE DOSAGE DESDITS ANTICORPS ET CELLULE INÉDITE GÉNÉTIQUEMENT MODIFIÉE UTILISABLE DANS LE CADRE DUDIT BIODOSAGE OU AVEC LEDIT NÉCESSAIRE DE DOSAGE

(30) Priority: 30.06.2009 JP 2009155183
(43) Date of publication of application: 09.05.2012
(60) Divisional application: 14195873.6
(73) Proprietor: Otsuka Pharmaceutical Co., Ltd., Tokyo 101-8535 (JP)
(72) Inventor: ARAKI, Naohiro, Osaka-shi Osaka 541-0045 (JP)
(74) Representative: Von Kreisler Selting Werner - Partnerschaft von Patentanwälten und Rechtsanwälten mbB
(86) International application number: PCT/JP2010/060731
(87) International publication number: WO 2011/001885

(56) References cited:
- WO-A1-2007/139080
- WO-A1-2007/142208
- WO-A1-2009/045292
- JP-A- 2006 204 225
- JP-T- 2001 518 309
- JP-T- 2005 507 245
- JP-T- 2007 531 514
- TITUS STEVEN A ET AL: "Cell-based PDE4 assay in 1536-well plate format for high-throughput screening", JOURNAL OF BIOMOLECULAR SCREENING, vol. 13, no. 7, August 2008 (2008-08), pages 609-618, XP002689797, ISSN: 1087-0571
- MICHEL ATGER ET AL.: 'Autoantibodies interacting with purified native thyrotropin receptor' EUR. J. BIOCHEM. vol. 265, 1999, pages 1022 - 1031, XP055076564

## Description

### Technical Field

The present invention relates to a kit comprising a cell expressing a thyroid stimulating hormone receptor (TSHR), a cAMP dependent calcium channel and a calcium sensitive protein, and to methods for the diagnosis of thyroid disease and for diagnosis of hypothyroidisms using the cell.

### Background Art

A thyroid stimulating hormone (TSH) produced by the pituitary gland binds to a thyroid stimulating hormone receptor (TSHR) present in the thyroid gland to promote the secretion of a thyroid hormone. Since the thyroid hormone is a hormone that enhances systemic metabolism, abnormal increase or abnormal decrease in the action of this hormone variously affects the mind and body, causing thyroid disease. For example, regarding Graves' disease, a thyroid stimulating antibody (TSAb) is produced in the body, and this antibody, instead of TSH, overstimulates TSHR so that the functions of the thyroid gland are increased and symptoms such as enlargement of the thyroid gland, exophthalmos and tachycardia appear. On the other hand, among hypothyroidisms, there are some diseases in which a thyroid stimulation blocking antibody (TSBAb) is produced so that the functions of the thyroid gland are decreased and symptoms such as a weight gain, depression, and general fatigue appear.

So far, the assay of these autoantibodies (TSAb and TSBAb) contained in blood has been used for the diagnosis of Graves' disease and hypothyroidism.

The representative examples of a method for measuring the autoantibodies have been reported as follows:
a radioreceptor assay method (TBII method) using a radioisotope-labeled TSH or a monoclonal antibody against TSHR, in which said radioisotope-labeled TSH or said monoclonal antibody against TSHR competitively inhibits the binding of the autoantibody in a patient's serum to TSHR so that an amount of the bound autoantibody can be measured; and
a bioassay method (TSAb method) for measuring an amount of TSAb, in which porcine thyroid gland cells are treated with an antibody that binds to TSHR and the amount of TSAb is determined by measuring an increase in the concentration of cAMP in the thyroid gland cells using radioisotope-labeled cAMP (Non Patent Literatures 1 and 2).

WO 2009/045292 discloses an assay for TSH-receptor autoantibodies based on TSHR transfected cells (i.a. CHO cells). These assays use cAMP-induced luciferase levels as endpoint.
Non Patent Literature 1: Methods in Enzymology, 74, 405-420 (1981)
Non Patent Literature 2: J Clin Endocrinol Metab. 1986 May; 62 (5): 855-62

### Summary of Invention

### Technical Problem to be solved

An object of the present invention is to provide a composition for measurement of TSAb and/or TSBAb and a composition for diagnosis of thyroid disease, which can be manipulated more conveniently than conventional methods without a use of a radioisotope that requires special techniques or equipment. Moreover, a further object of the present invention is to provide a method for diagnosing thyroid disease using the composition.

### Solution to Problem

The present inventor used a calcium sensitive protein to measure the amount of calcium ion entry into a cell stimulated by cAMP which is formed by the binding of a thyroid stimulating antibody (TSAb) to a thyroid stimulating hormone receptor (TSHR). As a result, the present inventor has successfully measured the amount of TSAb without using a radioisotope. Furthermore, the present inventor has also successfully measured the amount of a thyroid stimulation blocking antibody (TSBAb) using similar principles, and consequently completed the present invention.

Specifically, the present invention relates to a kit for diagnosing thyroid disease, comprising a cell expressing a thyroid stimulating hormone receptor (TSHR), a cAMP dependent calcium channel and a calcium sensitive protein, wherein the calcium sensitive protein is a protein that emits luminescence in response to calcium. Furthermore, the present invention relates to a method for diagnosing thyroid disease comprising the following steps (1), (2), and (3) or (1'), (2), and (3) :
(1) preparing a mixture containing a cell comprised in the kit as defined above, a luminescent substrate for the calcium sensitive protein, a Ca²⁺-free medium or a Ca²⁺/Mg²⁺-free medium, TSH and a sample derived from the blood of a test subject; or (1') preparing a mixture containing a cell comprised in the kit as defined above, a luminescent substrate for the calcium sensitive protein, a Ca²⁺-free medium or a Ca²⁺/Mg²⁺-free medium and a sample derived from the blood of a test subject;
(2) adding a Ca²⁺-containing solution to the mixture prepared in (1) or (1'); and
(3) measuring the luminescence of the calcium sensitive protein emitted from the cell.

Furthermore, the present invention also relates to a method for diagnosing hypothyroidism, comprising the following steps (1) to (3):
(1) preparing a mixture containing a cell comprised in the kit as defined above, a luminescent substrate for the calcium sensitive protein, a Ca²⁺-containing medium, TSH and a sample derived from the blood of a test subject;
(2) adding a forskolin-containing solution to the mixture prepared in (1); and
(3) measuring the luminescence of the calcium sensitive protein emitted from the cell.

### Advantageous Effects of Invention

The present invention eliminates the need of complicated procedures accompanied with use of radioisotopes. Thus, the measurement of the amount of a thyroid stimulating antibody (TSAb) and the amount of a thyroid stimulation blocking antibody (TSBAb) contained in a biological sample and the diagnosis of thyroid disease can be achieved by simple and safe procedures.

### Brief Description of Drawings

[Figure 1] Figure 1 shows that luminescence emitted from CHO cells expressing human TSHR, modified CNG channel and modified aequorin is dependent on a concentration of bovine TSH (bTSH).
[Figure 2] Figure 2 shows an amount of luminescence emitted from the CHO cells expressing human TSHR, modified CNG channel and modified aequorin, in the presence of a low dose of bTSH.
[Figure 3] Figure 3 shows effects of a concentration of a luminescent substrate for the aequorin and the incubation time on an amount of luminescence emitted from the CHO cells expressing human TSHR, modified CNG channel and modified aequorin.
[Figure 4] Figure 4 shows effects of an amount of introduced TSHR expression plasmid on an amount of luminescence emitted from the CHO cells expressing human TSHR, modified CNG channel and modified aequorin.
[Figure 5] Figure 5 shows a relationship between a concentration of CHO cells expressing human TSHR, modified CNG channel and modified aequorin, and an amount of luminescence from the cells.
[Figure 6] Figure 6 shows a relationship between a concentration of CHO cells expressing human TSHR, modified CNG channel and modified aequorin, and an amount of luminescence from the cells, where the amount of luminescence is indicated as a relative value which is calculated on the assumption that a relative value for each blank is 1.
[Figure 7] Figure 7 shows effects of a concentration of added CaCl₂ on an amount of luminescence emitted from the CHO cells expressing human TSHR, modified CNG channel and modified aequorin.
[Figure 8] Figure 8 shows that a kit according to the present invention is capable of quantifying a thyroid stimulating antibody (TSAb).
[Figure 9] Figure 9 shows that a kit according to the present invention is capable of detecting a thyroid stimulation blocking antibody (TSBAb).
[Figure 10] Figure 10 shows that a kit according to the present invention utilizes cell desensitization to be capable of detecting a thyroid stimulation blocking antibody (TSBAb) .
[Figure 11] Figure 11 shows that a kit according to the present invention is capable of detecting a thyroid stimulating antibody (TSAb) with higher sensitivity compared to a conventional product (thyroid stimulating autoantibody kit; TSAb kit "YAMASA" (R)).
[Figure 12] Figure 12 shows the time course of an amount of luminescence emitted from the CHO cells expressing human TSHR, modified CNG channel and modified aequorin.
[Figure 13] Figure 13 shows a concentration dependence of a blocking antibody.
[Figure 14] Figure 14 shows that addition of forskolin solution allows a blocking antibody to be detected in a dose-dependent manner.
[Figure 15] Figure 15 shows change in an amount of luminescence depending on incubation time with a stimulating antibody (TSAb).
[Figure 16] Figure 16 shows change in an amount of luminescence depending on incubation time with a blocking antibody (TSBAb).
[Figure 17] Figure 17 shows change in an amount of luminescence induced by addition of forskolin depending on incubation time with a blocking antibody (TSBAb).
[Figure 18] Figure 18 shows a plasmid pmCNGa2.
[Figure 19] Figure 19 shows a plasmid pcDNA mt sAEQ.
[Figure 20] Figure 20 shows a histogram of the TSAb values of 48 normal individuals measured by means of a kit according to the present invention.
[Figure 21] Figure 21 shows distribution of TSAb values in serum samples derived from various thyroid diseases measured by means of a kit according to the present invention.

### Description of Embodiments

Disclosed is a cell expressing a thyroid stimulating hormone receptor (TSHR), a cAMP dependent calcium channel and a calcium sensitive protein, wherein the calcium sensitive protein is a protein that emits luminescence in response to calcium.

The thyroid stimulating hormone receptor (TSHR) can be any receptor to which a thyroid stimulating hormone (TSH) binds, and includes receptors that activate adenylate cyclase to increase cAMP. The origin of TSHR is not particularly limited as long as it is a mammal. The origin can be, for example, a human, a mouse, bovine, a rat or a pig. Moreover, TSHR may have one or several amino acids modified (added, substituted, deleted) appropriately in the amino acid sequence, or TSHR may be a protein that consists of an amino acid sequence having 70% or more, 80% or more, 85% or more, 90% or more, 95% or more, 98% or more, or 99% or more homology to the amino acid sequence of natural TSHR; binds to TSH; and has the function of increasing cAMP through activation of adenylate cyclase. TSHR can be a protein having the amino acid sequence represented by SEQ ID NO: 1. Furthermore, TSHR can be a protein that consists of an amino acid sequence having 70% or more, 80% or more, 85% or more, 90% or more, 95% or more, 98% or more, or 99% or more homology to the amino acid sequence represented by SEQ ID NO: 1; binds to TSH; and has the function of increasing cAMP through activation of adenylate cyclase.
Alternatively, TSHR can be a chimeric protein of TSHR with a receptor analogous to TSHR, for example, a corpus luteum hormone receptor, a follicle stimulating hormone receptor or a human chorionic gonadotropin receptor. These chimeric proteins may be prepared by substituting a portion other than amino acid residues 8-89 or 8-165 in TSHR with an appropriate portion of the corpus luteum hormone receptor, the follicle stimulating hormone receptor or the human chorionic gonadotropin receptor. For example, for preparing the TSHR-corpus luteum hormone receptor chimeric protein, amino acid residues 90-165 in TSHR may be substituted with the segment Mc2 of an LH-CG receptor, and amino acid residues 261-370 in the TSHR may further be substituted with the segment Mc4 of the LH-CG receptor.
As used herein, TSH is not particularly limited as long as it is derived from a mammal. TSH can be derived from, for example, a human, a mouse, bovine, a rat, or a pig. TSH may have one or several amino acids modified (added, substituted, deleted) appropriately in the amino acid sequence, or may be a protein that consists of an amino acid sequence having 70% or more, 80% or more, 85% or more, 90% or more, 95% or more, 98% or more, or 99% or more homology to the amino acid sequence of natural TSH; binds to TSHR; and has the function of increasing cAMP through activation of adenylate cyclase.

The cAMP dependent calcium channel is a channel that changes the amount of calcium ion entry into a cell in response to change in the concentration of cAMP, and includes channels that increase the amount of calcium ion entry into a cell in response to increase in the concentration of cAMP. Examples of the cAMP dependent calcium channel include a CNG (cyclic nucleotide gated ion channel) calcium channel. Optionally, the cAMP dependent calcium channel may have one or several amino acids modified (added, substituted, deleted) in the amino acid sequence and may be modified (including substituted, added, and deleted) such that it exhibits, for example, higher sensitivity to cAMP than cGMP. The CNG calcium channel can be a protein that consists of an amino acid sequence having 70% or more, 80% or more, 85% or more, 90% or more, 95% or more, 98% or more, or 99% or more homology to the amino acid sequence of the natural CNG calcium channel and increases the amount of calcium ion entry into a cell in response to increase in the concentration of cAMP. Examples of the modification include the substitution of the 460th cysteine in a mouse CNG calcium channel with tryptophan, the substitution of the 583rd glutamic acid in a mouse CNG calcium channel with methionine, the substitution of the 537th threonine in a bovine CNG calcium channel with serine, methionine, valine, or alanine, and combinations thereof. These substitutions exemplified above are not limited to the animal species in which the substitutions are found respectively, and are also applicable to amino acid substitution at corresponding sites in other animal species. For example, threonine in a mouse CNG calcium channel corresponding to the 537th threonine in the bovine CNG calcium channel can be substituted with serine, methionine, valine, or alanine. Such substitution can be performed at one or more position(s). For example, the substitution of the 460th cysteine in the mouse CNG calcium channel with tryptophan is performed, and the substitution of the 583rd glutamic acid with methionine can also be performed. The CNG calcium channel can consist of an α-subunit and/or a β-subunit. It may be of any constitution, for example, consisting of at least one subunit selected from the group consisting of an α2 subunit, an α3 subunit, an α4 subunit, and a β1b subunit. Furthermore, the subunit may be modified as described above.
The origin of the CNG calcium channel is not particularly limited as long as it is a mammal. The origin can be, for example, a human, a mouse, bovine, a rat or a pig. The CNG calcium channel can be a protein having the amino acid sequence represented by SEQ ID NO: 2. Furthermore, the CNG calcium channel can be a protein that consists of an amino acid sequence having 70% or more, 80% or more, 85% or more, 90% or more, 95% or more, 98% or more, or 99% or more homology to the amino acid sequence represented by SEQ ID NO: 2 and increases the amount of calcium ion entry into a cell in response to increase in the concentration of cAMP.

The calcium sensitive protein is a protein whose structure is changed in response to calcium and that emits luminescence in response to calcium.
Examples of the calcium sensitive protein include aequorin, cameleon (Invitrogen Corp.), Casel2 (Evrogen), clytin, obelin, mitrocomin, mineopsin, berovin, a protein comprising two GFPs differing in color, bound to calcium sensitive calmodulin and a partial sequence of myosin light chain kinase binding thereto, a calcium sensitive protein comprising calmodulin bound to between the 144th and 146th residues in the amino acid sequence of GFP, and a protein of probe No. G3-85 or Al-2 described in Japanese Patent Laid-Open No. 2002-153279, and apoproteins thereof, if any, (e.g., apoaequorin).
The amino acid sequence of the calcium sensitive protein may be modified (added, substituted, deleted) appropriately according to the purpose or may be modified to increase the amount of luminescence and/or to improve an SN ratio. The modification includes the addition, substitution, and deletion of one or several amino acids in the amino acid sequence. The calcium sensitive protein includes proteins that consist of an amino acid sequence having 70% or more, 80% or more, 85% or more, 90% or more, 95% or more, 98% or more, or 99% or more homology to the amino acid sequence of the natural calcium sensitive protein and emit luminescence in response to calcium. For example, the calcium sensitive protein may be modified such that its gene is optimized for human codon usage and it has a mitochondrial targeting signal.
The calcium sensitive protein can be a protein having the amino acid sequence represented by SEQ ID NO: 3. Furthermore, the calcium sensitive protein can be a protein that consists of an amino acid sequence having 70% or more, 80% or more, 85% or more, 90% or more, 95% or more, 98% or more, or 99% or more homology to the amino acid sequence represented by SEQ ID NO: 3 and emits luminescence in response to calcium.

The cell expressing a thyroid stimulating hormone receptor (TSHR), a cAMP dependent calcium channel and a calcium sensitive protein transiently or stably expresses each of the thyroid stimulating hormone receptor (TSHR), the cAMP dependent calcium channel and the calcium sensitive protein. The cell is not particularly limited and can be a cell line such as a CHO cell, a HEK293 cell, or a 3T3 cell. For example, the cell expressing a thyroid stimulating hormone receptor (TSHR), a cAMP dependent calcium channel and a calcium sensitive protein can be a CHO cell stably expressing each protein, wherein TSHR has the amino acid sequence represented by SEQ ID NO: 1, the cAMP dependent calcium channel is a modified CNG calcium channel having the amino acid sequence represented by SEQ ID NO: 2 and the calcium sensitive protein is modified apoaequorin having the amino acid sequence represented by SEQ ID NO: 3. Furthermore, the cell expressing a thyroid stimulating hormone receptor (TSHR), a cAMP dependent calcium channel and a calcium sensitive protein can be, for example, a CHO cell stably expressing TSHR, the cAMP dependent calcium channel and the calcium sensitive protein, each of which is a protein that consists of an amino acid sequence having 70% or more, 80% or more, 85% or more, 90% or more, 95% or more, 98% or more, or 99% or more homology to the amino acid sequence of any one of SEQ ID NOs: 1-3 and maintains the functions of the thyroid stimulating hormone receptor (TSHR), the cAMP dependent calcium channel or the calcium sensitive protein.
Moreover, a cell naturally expressing one or more protein(s) selected from the group consisting of the thyroid stimulating hormone receptor (TSHR), the cAMP dependent calcium channel and the calcium sensitive protein may be used. In this case, the cell expressing a thyroid stimulating hormone receptor (TSHR), a cAMP dependent calcium channel and a calcium sensitive protein can also be prepared by forcing the cell to transiently or stably express the protein(s) that are not expressed in the cell. Examples of such cell include a thyroid gland-derived cell endogenously expressing the TSHR (e.g., FRTL-5 or Nthy-ori 3-1) that is forced to transiently or stably express each of the cAMP dependent calcium channel and the calcium sensitive protein and an olfactory tissue-derived cell endogenously expressing the CNG calcium channel that is forced to transiently or stably express each of the TSHR and the calcium sensitive protein.

The cell expressing a thyroid stimulating hormone receptor (TSHR), a cAMP dependent calcium channel and a calcium sensitive protein can be cryopreserved. The cryopreservation can be performed at an appropriate temperature, for example, -20°C or -80°C, in a cell cryopreservation solution. The cell cryopreservation solution is not limited and includes CELLBANKER (R) (Nippon Zenyaku Kogyo Co., Ltd.), BAMBANKER (R) (Lymphotec Inc.), Cellvation (R) (CELOX LABORATORIES, Inc.), CryoStor (R) (BIOLIFE SOLUTIONS Ltd.). Cells can be cryopreserved in a large number of the same lot so that a cell-derived measurement error among compositions or kits is drastically reduced. As a result, the reproducibility of measurement results can be improved. Moreover, the cell expressing a thyroid stimulating hormone receptor (TSHR), a cAMP dependent calcium channel and a calcium sensitive protein maintains sensitivity sufficient for detecting a thyroid stimulating antibody (TSAb) and a thyroid stimulation blocking antibody (TSBAb) present in human blood, even after being cryopreserved and thawed using a warm bath. Moreover, after thawing, the cell is merely transferred into an appropriate container and cultured for approximately 2 hours, and the state of the cell is not deteriorated by a reagent added for detecting TSAb and TSBAb, or by human blood-derived components.

The disclosed composition comprising the cell expressing a thyroid stimulating hormone receptor (TSHR), a cAMP dependent calcium channel and a calcium sensitive protein (e.g., an aqueous solution comprising the cell expressing a thyroid stimulating hormone receptor (TSHR), a cAMP dependent calcium channel and a calcium sensitive protein) can be used for assaying the amount of a thyroid stimulating antibody and/or the amount of a thyroid stimulation blocking antibody, for diagnosing thyroid disease, for determining a human having a high risk of developing thyroid disease, and/or for determining therapeutic effect on a human under treatment of thyroid disease. As used herein, the thyroid disease includes hyperthyroidism and hypothyroidism. The hyperthyroidism includes Graves' disease, and the hypothyroidism includes Hashimoto's disease. As used herein, the Hashimoto's disease includes hypothyroidism that is TSBAb-positive in blood, atrophic thyroiditis free from goiter, atrophic thyroiditis caused by TSBAb and myxedema.
The cell expressing a thyroid stimulating hormone receptor (TSHR), a cAMP dependent calcium channel and a calcium sensitive protein is as described above.
The thyroid stimulating antibody (TSAb) is an antibody capable of acting as a TSHR agonist and can be found in the blood of a Graves' disease patient.
The thyroid stimulation blocking antibody (TSBAb) is an antibody capable of binding to TSHR and acting as a TSHR antagonist and includes, for example, antibodies that competitively inhibit the binding of TSH to TSHR. The thyroid stimulation blocking antibody (TSBAb) can be found in the blood of a hypothyroidism patient, for example, in the blood of a Hashimoto's disease patient.

The thyroid stimulating antibody (TSAb) or the thyroid stimulation blocking antibody (TSBAb) present in a biological sample can be measured by use of the following mechanism of action:
(1) When thyroid stimulating antibody (TSAb) is present in biological sample TSAb increases cAMP through the action on TSHR of the above cell. As a result, the CNG calcium channel is activated so that calcium entry into the cell is increased. Thus, the calcium sensitive protein emits luminescence. This means that the presence of TSAb in the sample is represented by the luminescence of the calcium sensitive protein as an output.
(2) When thyroid stimulation blocking antibody (TSBAb) is present in biological sample
   (a) Method using competitive inhibition against TSH Upon addition together with TSH, TSBAb competitively inhibits the binding of the TSH to TSHR. As a result, the action of TSH is inhibited to prevent increase in the concentration of cAMP and thereby also prevent CNG calcium channel-mediated increase in calcium entry. Thus, the luminescence of the calcium sensitive protein is reduced. This means that the presence of TSBAb in the sample is represented by the suppression of luminescence of the calcium sensitive protein as an output.
   (b) Method using desensitization of CNG calcium channel Upon addition together with TSH, TSBAb competitively inhibits the binding of the TSH to TSHR. As a result, the action of TSH is inhibited to prevent increase in the concentration of cAMP. In this case, if TSBAb is absent, the concentration of cAMP is increased by the action of TSH to activate the CNG calcium channel. However, after the given time, the CNG calcium channel is desensitized and thus, does not respond to newly added forskolin (or agent increasing the concentration of cAMP). Accordingly, if TSBAb is absent in the sample, calcium entry into the cell does not occur. This means that the absence of TSBAb is represented by the suppression of luminescence of the calcium sensitive protein as an output. On the other hand, if TSBAb is present in the sample, the CNG calcium channel is not desensitized. Thus, the luminescence of the calcium sensitive protein is not reduced.

By using the above composition, based on the mechanism of action described above, a thyroid stimulating antibody and/or a thyroid stimulation blocking antibody in a biological sample can be detected; the concentrations of a thyroid stimulating antibody and/or a thyroid stimulation blocking antibody between two biological samples can be compared; or the relative amounts of a thyroid stimulating antibody and/or a thyroid stimulation blocking antibody in two biological samples can be measured. Furthermore, by using of the above composition the concentrations of a thyroid stimulating antibody and/or a thyroid stimulation blocking antibody in a biological sample can also be measured.
As used herein, the biological sample includes organism-derived samples such as blood and a sample prepared from blood and includes, for example, human blood, a sample prepared from human blood, canine blood, a sample prepared from canine blood, feline blood, and a sample prepared from feline blood.

By using the above composition, one can measure a thyroid stimulating antibody and/or a thyroid stimulation blocking antibody in human blood. Thus, a test subject can be diagnosed as having thyroid disease or not.
When the mechanism of action described above in (1) is used, Graves' disease can be diagnosed using the above composition. For example, Graves' disease can be diagnosed by measuring the amount of luminescence of the calcium sensitive protein emitted from the cell treated with a blood sample of a test subject and the amount of luminescence of the calcium sensitive protein emitted from the cell treated with the same amount of a blood sample (standard) of a normal individual as that of the blood sample of the test subject. In this case, when the amount of luminescence emitted from the cell treated with the blood sample of the test subject is higher than that emitted from the cell treated with the blood sample (standard) of the normal individual, the serum concentration of a thyroid stimulating antibody (TSAb) of the test subject can be determined to be higher than that of the normal individual. Thus, the test subject can be diagnosed as Graves' disease.
Moreover, the respective amounts of luminescence (integrated values) emitted from the cells treated with blood samples of a large population of normal individuals, for example, 50 to 100 normal individuals, are measured in advance, and a mean thereof and standard deviation (SD) may be calculated. When the amount of luminescence emitted from the cell treated with a blood sample of a test subject is higher than the mean + nSD (e.g., n = 1, 2, 3, 4, or 5) of the amounts of luminescence (integrated values) emitted from the cells treated with the blood samples of the normal individual population, the serum concentration of a thyroid stimulating antibody of the test subject can be determined to be higher than that of the normal individuals. Thus, the test subject can be diagnosed as Graves' disease.
In the present specification and claims, a value obtained from the "sample derived from the blood of a normal individual" can be, for example, a measured value of a blood sample (standard) of a normal individual or a value obtained in advance from measured values of a normal individual population, unless otherwise specified.

Alternatively, the amount of luminescence (integrated value) emitted from the cell treated with a blood sample of a test subject / the amount of luminescence (integrated value) emitted from the cell treated with a blood sample of a normal individual × 100 (%) may be calculated (hereinafter, this calculated value is defined as a calculated value A). The respective amounts of luminescence (integrated values) emitted from the cells treated with respective blood samples of a large population of normal individuals and a large population of untreated Graves' disease patients (e.g., 50 to 100 individuals per population) are measured in advance, and a cutoff value may be set such that a true positive rate of Graves' disease and/or a true negative rate (i.e., being a normal individual) are, for example, 80% or more, 90% or more, 92% or more, 94% or more, 96% or more, or 98% or more, respectively. When the calculated value A is higher than the cutoff value, the test subject can be diagnosed as Graves' disease. The cutoff value can be set appropriately depending on the properties of a target population, such as age and sex and can be set to, for example, 120%, 130%, 140%, 150%, 160%, 170%, 180%, 190%, or 200%.
Furthermore, an antibody (TSAb) standard (e.g., NIBSC 90/672 or 65/122) is also used, and its concentration may be serially diluted to prepare a calibration curve. With reference to the calibration curve, the actual serum concentration of an antibody (TSAb) is calculated from the measured amount of luminescence of the calcium sensitive protein emitted from the cell treated with a blood sample of a test subject. This concentration can be compared with the previously measured serum concentration of an antibody (TSAb) in each of a large population of normal individuals and a large population of untreated Graves' disease patients (e.g., 50 to 100 individuals per population), or known data reported in a document to diagnose the test subject as being Graves' disease or not. For example, when the concentration is higher than the mean + nSD (e.g., n = 1, 2, 3, 4, or 5) of the serum concentrations of (TSAb) of the normal individual population, the test subject can be diagnosed as Graves' disease.
For the measurement of the amount of luminescence, it is preferred to measure the integrated value for the given time.
For example, the integrated value can be measured for 5 s, 10 s, 15 s, 20 s, 30 s, 40 s, 50 s, or 1 min.

Moreover, when the mechanism of action described above in (2)(a) is utilized, hypothyroidism (including Hashimoto's disease) can be diagnosed using the above composition. For example, hypothyroidism can be diagnosed by measuring the amount of TSH-induced luminescence of the calcium sensitive protein in the cell treated with a blood sample of a test subject and the amount of TSH-induced luminescence of the calcium sensitive protein in the cell treated with the same amount of a blood sample (standard) of a normal individual as that of the blood sample of the test subject. In this case, when the amount of luminescence emitted from the cell treated with the blood sample of the test subject is lower than that emitted from the cell treated with the blood sample (standard) of the normal individual, the serum concentration of a thyroid stimulation blocking antibody (TSBAb) of the test subject can be determined to be higher than that of the normal individual. Thus, the test subject can be diagnosed as hypothyroidism.
Moreover, the respective amounts of TSH-induced luminescence (integrated values) of the calcium sensitive proteins in the cells treated with blood samples of a large population of normal individuals, for example, 50 to 100 normal individuals, are measured in advance, and a mean thereof and standard deviation (SD) may be calculated. When the amount of TSH-induced luminescence from the cell treated with a blood sample of a test subject is lower than the mean - nSD (e.g., n = 1, 2, 3, 4, or 5) of the amounts of TSH-induced luminescence (integrated values) from the cells treated with the blood samples of the normal individual population, the serum concentration of a thyroid stimulation blocking antibody (TSBAb) in the test subject can be determined to be higher than that in the normal individuals. Thus, the test subject can be diagnosed as hypothyroidism.
Alternatively, the amount of luminescence (integrated value) emitted from the cell treated with a blood sample of a test subject / the amount of luminescence (integrated value) emitted from the cell treated with a blood sample of a normal individual × 100 (%) may be calculated (hereinafter, this calculated value is defined as a calculated value B). The respective amounts of luminescence (integrated values) attributed to respective blood samples of a large population of normal individuals and a large population of untreated hypothyroidism patients (e.g., 50 to 100 individuals per population) are measured in advance, and a cutoff value is set such that a true positive rate of hypothyroidism and/or a true negative rate (i.e., being a normal individual) are, for example, 80% or more, 90% or more, 92% or more, 94% or more, 96% or more, or 98% or more, respectively. When the calculated value B is lower than the cutoff value, the test subject can also be diagnosed as hypothyroidism. The cutoff value can be set appropriately depending on the properties of a target population, such as age and sex and can be set to, for example, 10%, 20%, 30%, 40%, 50%, 60%, 70%, 80%, or 90%.

Furthermore, an antibody (TSBAb) standard having a known concentration is also used, and its concentration may be serially diluted to prepare a calibration curve. With reference to the calibration curve, the actual serum concentration of an antibody (TSBAb) is calculated from the measured amount of TSH-induced luminescence of the calcium sensitive protein in the cell treated with a blood sample of a test subject. This concentration can be compared with the previously measured serum concentration of an antibody (TSBAb) in each of a large population of normal individuals and a large population of untreated hypothyroidism patients (e.g., 50 to 100 individuals per population), or known data reported in a document to diagnose the test subject as hypothyroidism or not. For example, when the concentration is higher than the mean + nSD (e.g., n = 1, 2, 3, 4, or 5) of the serum concentrations of (TSBAb) of the normal individual population, the test subject can be diagnosed as hypothyroidism.
For the measurement of the amount of luminescence, it is preferred to measure the integrated value for the given time. For example, the integrated value can be measured for 5 s, 10 s, 15 s, 20 s, 30 s, 40 s, 50 s, or 1 min.
Moreover, when the mechanism of action described above in (2)(b) is utilized, hypothyroidism (including Hashimoto's disease) can be diagnosed using the above composition. For example, hypothyroidism can be diagnosed by measuring the amount of forskolin-induced luminescence of the calcium sensitive protein in the cell treated with a blood sample of a test subject and the amount of forskolin-induced luminescence of the calcium sensitive protein in the cell treated with the same amount of a blood sample (standard) of a normal individual as that of the blood sample of the test subject. In this case, when the amount of luminescence emitted from the cell treated with the blood sample of the test subject is higher than that emitted from the cell treated with the blood sample (standard) of the normal individual, the serum concentration of a thyroid stimulation blocking antibody (TSBAb) of the test subject can be determined to be higher than that of the normal individual. Thus, the test subject can be diagnosed as hypothyroidism.
Moreover, the respective amounts of forskolin-induced luminescence (integrated values) of the calcium sensitive proteins in the cells treated with blood samples of a large population of normal individuals, for example, 50 to 100 normal individuals, are measured in advance, and a mean thereof and standard deviation (SD) may be calculated. When the amount of forskolin-induced luminescence from the cell treated with a blood sample of a test subject is higher than the mean + nSD (e.g., n = 1, 2, 3, 4, or 5) of the amounts of forskolin-induced luminescence (integrated values) from the cells treated with the blood samples of the normal individual population, the serum concentration of a thyroid stimulation blocking antibody (TSBAb) of the test subject can be determined to be higher than that of the normal individuals. Thus, the test subject can also be diagnosed as hypothyroidism.

Alternatively, the amount of luminescence (integrated value) emitted from the cell treated with a blood sample of a test subject / the amount of luminescence (integrated value) emitted from the cell treated with a blood sample of a normal individual × 100 (%) may be calculated (hereinafter, this calculated value is defined as a calculated value C). The respective amounts of luminescence (integrated values)
attributed to respective blood samples of a large population of normal individuals and a large population of untreated hypothyroidism patients (e.g., 50 to 100 individuals per population) are measured in advance, and a cutoff value is set such that a true positive rate of hypothyroidism and/or a true negative rate (i.e., being a normal individual) are, for example, 80% or more, 90% or more, 92% or more, 94% or more, 96% or more, or 98% or more, respectively. When the calculated value C is higher than the cutoff value, the test subject can also be diagnosed as hypothyroidism. The cutoff value can be set appropriately depending on the properties of a target population, such as age and sex and can be set to, for example, 150%, 200%, 300%, 400%, 500%, 600%, 700%, 800%, or 900%.
Furthermore, an antibody (TSBAb) standard having a known concentration is also used, and its concentration may be serially diluted to prepare a calibration curve. The actual serum concentration of an antibody (TSBAb) is calculated from the measured forskolin-induced luminescence of the calcium sensitive protein in the cell treated with a blood sample of a test subject. This concentration can be compared with the previously measured serum concentration of an antibody (TSBAb) in each of a large population of normal individuals and a large population of untreated hypothyroidism patients (e.g., 50 to 100 individuals per population), or known data reported in a document to diagnose the test subject as hypothyroidism or not. For example, when the concentration is higher than the mean + nSD (e.g., n = 1, 2, 3, 4, or 5) of the serum concentrations of (TSBAb) of the normal individual population, the test subject can be diagnosed as hypothyroidism.
For the measurement of the amount of luminescence, it is preferred to measure the integrated value for the given time.
For example, the integrated value can be measured for 5 s, 10 s, 15 s, 20 s, 30 s, 40 s, 50 s, or 1 min.

Moreover, a human having a high risk of developing thyroid disease, for example, a human having a high risk of developing Graves' disease or hypothyroidism, can be determined using the above composition.
For example, when the serum concentration of a thyroid stimulating antibody measured using the above composition in medical examination is lower than the numeric value of a Graves' disease patient and higher than the numeric value of a normal individual, this person can be determined as a human having a high risk of developing Graves' disease. When the serum concentration of a thyroid stimulation blocking antibody is lower than the numeric value of hypothyroidism and higher than the numeric value of a normal individual, this person can be determined as a human having a high risk of developing hypothyroidism. Moreover, when the serum concentration of a thyroid stimulating antibody measured using the above composition in medical examination is gradually increased over time, this person can be determined as a human having a high risk of developing Graves' disease. When the serum concentration of a thyroid stimulation blocking antibody is gradually increased over time, this person can be determined as a human having a high risk of developing hypothyroidism.

Furthermore, the effectiveness of treatment for a human under treatment of thyroid disease, for example, a human with Graves' disease or hypothyroidism under treatment thereof, can also be determined using the above composition.
For example, the concentration of a thyroid stimulating antibody or a thyroid stimulation blocking antibody of blood samples collected from the same individual both before and after treatment, and time-dependent change in the concentration can be measured using the above composition to determine the presence or absence of effectiveness of the treatment. When the serum concentration of the thyroid stimulating antibody measured using the above composition is lower after treatment than before treatment, the treatment of Graves' disease can be determined as being effective. When the serum concentration of the thyroid stimulation blocking antibody measured using the above composition is lower before treatment than after treatment, the treatment of hypothyroidism can be determined as being effective.

The present invention provides a kit that comprises the cell expressing a thyroid stimulating hormone receptor (TSHR), a cAMP dependent calcium channel and a calcium sensitive protein and can further comprise at least one selected from the group consisting of a medium for cell culture, a detection solution, a luminescent substrate for the calcium sensitive protein or an aqueous solution thereof, an antibody separation solution, a plate for cell culture or a test tube, TSH or an aqueous solution thereof, an anti-TSH antibody and a normal human IgG control serum. For example, each substance constituting the kit are individually packaged, and these packages can be placed together in one container such as a box to prepare a kit. The cell expressing a thyroid stimulating hormone receptor (TSHR), a cAMP dependent calcium channel and a calcium sensitive protein can be prepared appropriately and is prepared, for example, at a concentration of 3 × 10⁴ cells/ml to 3 × 10⁶ cells/ml. The cell may be prepared, for example, by suspending it at a concentration of 3 × 10⁶ cells/ml in an aqueous solution. When a 96-well plate is used as a plate for cell culture, the cell can be plated at a concentration of, for example, 3 × 10³ cells to 3 × 10⁵ cells per well of the 96-well plate. The medium for cell culture is not limited as long as it can maintain the cell. The medium for cell culture can be Ca²⁺-free or Ca²⁺/Mg²⁺-free.
The detection solution can contain CaCl₂, trypan blue, a cation that is capable of causing aequorin luminescence and can be substituted for calcium (e.g., a cadmium ion or a strontium ion), a magnesium ion, a zinc ion, a sulfuric acid ion and/or a carbonic acid ion. The concentrations of CaCl₂ and trypan blue can be adjusted appropriately by those skilled in the art. For example, the concentration of CaCl₂ is 9 to 18 mM, and the concentration of trypan blue is 0.001 to 0.010%. The detection solution is, for example, an aqueous solution containing 9 mM CaCl₂ and 0.002% trypan blue. The final concentration of CaCl₂ when the amount of luminescence of the calcium sensitive protein is measured can be set to 3 to 6 mM. Further, the detection solution can contain a cation that can be substituted for calcium, a magnesium ion, a zinc ion, a sulfuric acid ion and/or a carbonic acid ion, for example, by dissolving the cation that can be substituted for calcium, the magnesium ion, the zinc ion, the sulfuric acid ion and/or the carbonic acid ion in the detection solution.
The luminescent substrate for the calcium sensitive protein includes coelenterazine or a coelenterazine derivative that serves as a luminescent substrate for aequorin. The coelenterazine derivative includes ViviRen (R), (Promega Corp.). The concentration of ViviRen can be set appropriately and is, for example, 0.6 to 30 mM. The aqueous solution of the luminescent substrate for the calcium sensitive protein is, for example, an aqueous solution of 4 mM ViviRen (Promega Corp.).
The final concentration of ViviRen when the amount of luminescence of the calcium sensitive protein is measured can be set to 0.24 to 12 µM.
The antibody separation solution is not limited as long as a thyroid stimulating antibody and a thyroid stimulation blocking antibody can be collected from a blood sample. The antibody separation solution can contain 10 to 30% PEG6000. The antibody separation solution can be, for example, an aqueous solution containing 30% PEG6000.
Examples of the plate for cell culture include those allowing measurement of the amount of luminescence using a luminometer, for example, a 96-well plate allowing cell culture.
The test tube is not particularly limited and can be selected appropriately by those skilled in the art. For example, a test tube suitable for an apparatus for measuring luminescence emitted from the calcium sensitive protein can be used.
TSH is not limited as long as it can be used as a TSHR agonist. TSH may be used as a positive control. TSH can be, but not limited to, bovine TSH. TSH can be prepared appropriately by those skilled in the art and may be adjusted to 0.01 to 100 mU/ml. For example, bovine TSH is prepared as 1 mU/ml aqueous solution. The final concentration of bovine TSH when the amount of luminescence of the calcium sensitive protein is measured can be set to 0.6 µU/ml to 6 mU/ml. The final concentration of bovine TSH when the amount of luminescence of the calcium sensitive protein is measured is, for example, 100 µU/ml. Moreover, TSAb may be used instead of or in addition to TSH. TSAb can be a monoclonal antibody or a polyclonal antibody.
The normal human IgG control serum may be used as a negative control and can be prepared appropriately by those skilled in the art.

The anti-TSH antibody may be a polyclonal antibody or may be a monoclonal antibody. The anti-TSH antibody can be an antibody derived from an appropriate mammal and includes a mouse anti-TSH antibody, a rat anti-TSH antibody, a rabbit anti-TSH antibody and a goat anti-TSH antibody. The anti-TSH antibody may be modified appropriately by those skilled in the art. Moreover, TSH that serves as an antigen is also appropriately selected. TSH that serves as an antigen includes human TSH, mouse TSH, rat TSH, rabbit TSH, feline TSH and canine TSH. Examples of the anti-TSH antibody used in the kit of the present invention include a goat anti-human TSH polyclonal antibody. The concentration of the anti-TSH antibody can be set appropriately by those skilled in the art. For example, the concentration of an anti-TSH antibody solution packaged in the kit may be set to 0.01 µg/ml to 100 µg/ml. The final concentration of the anti-TSH monoclonal antibody when the amount of luminescence of the calcium sensitive protein is measured can be, for example, 0.05 to 5.48 µg/ml.
Among hypothyroidism patients, there is a patient whose amount of TSH in blood is a high value. In this case, the calcium sensitive protein in the cell emits luminescence due to the TSH. Thus, this patient may be diagnosed as Graves' disease though he or she is a hypothyroidism patient. However, a blood sample derived from the hypothyroidism patient having a high amount of TSH is added together with the anti-TSH antibody to the cell to neutralize the TSH derived from the patient. Thus, the incorrect diagnosis of the patient as Graves' disease can be avoided. Moreover, the amount of luminescence emitted from the calcium sensitive protein can be compared between the case where the anti-TSH antibody is added together with the patient-derived blood sample to the cell of the present invention and the case where the patient-derived blood sample is added to the cell of the present invention without the addition of the anti-TSH antibody, and thereby information about the amount of TSH of the blood of the patient can be obtained. A physician can more correctly diagnose thyroid disease by combining the information with clinical symptoms of the patient.

Moreover, the kit of the present invention further contains the serum of a thyroid disease patient, for example, the sera of a Graves' disease patient and/or a hypothyroidism patient. Such a serum can be used as a control or a standard for concentration calculation in the diagnosis of thyroid disease, in the assessment of a risk of developing the disease, or in the assessment of the effectiveness of treatment of the disease.

Furthermore, the kit of the present invention may contain a substance activating adenylate cyclase, for example, forskolin. The presence or absence of a thyroid stimulation blocking antibody in a sample derived from the blood of a test subject can be determined by adding the sample together with TSH to the cell, culturing the cell for the given time, and then adding forskolin thereto. Moreover, the concentration of a thyroid stimulation blocking antibody of the sample can also be measured. As a result, hypothyroidism (e.g., Hashimoto's disease) can be diagnosed, a human having a risk of developing hypothyroidism (e.g., Hashimoto's disease) can be determined, or therapeutic effect on a patient that has undergone the treatment of hypothyroidism (e.g., Hashimoto's disease) can be determined.

The kit according to the present invention is also useful for diagnostic aid that helps a physician to diagnose Graves' disease and/or hypothyroidism in view of clinical symptoms of the patient and/or other examination results. For example, the measurement of the amount of a thyroid stimulating antibody (TSAb) in a blood sample of a patient exhibiting hyperthyroidism using the composition or the kit according to the present invention can be useful for the differential diagnosis between Graves' disease and destructive hyperthyroidism (e.g., painless thyroiditis or subacute thyroiditis).

Also provided is a method for diagnosing Graves' disease, comprising the following steps (A) to (C):
(A) preparing a mixture containing a cell as defined above, a luminescent substrate for the calcium sensitive protein, a Ca²⁺-free medium and a sample derived from the blood of a test subject;
(B) adding a Ca²⁺-containing solution to the mixture prepared in (A); and
(C) measuring the luminescence of the calcium sensitive protein emitted from the cell.
The Ca²⁺-free medium used in the step (A) of the method can be selected appropriately by those skilled in the art and can be a Ca²⁺/Mg²⁺-free medium.
The Ca²⁺-containing solution used in the step (B) of the method can be selected appropriately by those skilled in the art and can be, for example, a CaCl₂ solution. The Ca²⁺-containing solution may further contain a cation that can be substituted for calcium (e.g., a cadmium ion or a strontium ion), a magnesium ion, a zinc ion, a sulfuric acid ion and/or a carbonic acid ion.
The mixture prepared in the step (A) of the method may further contain an anti-TSH antibody. The containing anti-TSH antibody in the mixture prepared in the step (A) of the method can avoid a hypothyroidism patient whose amount of TSH in blood is a high value from being incorrectly diagnosed as Graves' disease.
The order of addition of the substances described in the step (A) of the method can be set appropriately by those skilled in the art.
For example, a method for diagnosing Graves' disease is provided, which comprises the following steps:
(1) culturing a cell expressing a thyroid stimulating hormone receptor (TSHR), a cAMP dependent calcium channel and a calcium sensitive protein, in a Ca²⁺-free medium supplemented with a luminescent substrate for the calcium sensitive protein;
(2) adding a sample derived from the blood of a test subject to the cultured cell, which is further cultured; and
(3) adding a CaCl₂ solution to the cultured cell, and measuring the luminescence of the calcium sensitive protein emitted from the cell.
In the method, the cell may be cryopreserved. In this case, the cell can be thawed by mild operation such as a warm bath. The thawed cell may be cultured in a Ca²⁺/Mg²⁺-free medium supplemented with a luminescent substrate for the calcium sensitive protein. The luminescent substrate for the calcium sensitive protein includes coelenterazine and ViviRen (R).
The Ca²⁺-free medium is not limited as long as the cell can be maintained. For example, 130 mM NaCI, 5 mM KCl, 20 mM HEPES, 1 mM MgCl₂, 4.8 mM NaHCO₃, 5% PEG6000, pH 7.4 are used. Moreover, the Ca²⁺-free medium can be a Ca²⁺/Mg²⁺-free medium. The cell can be seeded at an appropriate concentration into an appropriate container and is seeded, for example, at a concentration of 3 to 30 × 10⁴ cells/ml and 90 µl/well into a 96-well plate compatible with a luminometer. The incubation time in the step (1) can be any time as long as it is equal to or longer than 2 hours. The incubation time can be set to 2-8 hours and is, for example, 3 hours. In general, for recovering from damage caused by subculture and increasing the amount of luminescence of the calcium sensitive protein such as aequorin, cells are usually cultured for approximately 12-24 hours after seeding the cells. In the present invention, it has been confirmed that even if the cells are cultured for approximately 2 hours, cell death caused by additives is not induced and the calcium sensitive protein exhibits strong luminescence. It has been confirmed that even approximately 2 hours after the cell seeding, strong luminescence from the calcium sensitive protein can be detected, for example, by employing aequorin as the calcium sensitive protein, optimizing its DNA sequence for human codon, and introducing a mitochondrial targeting signal, and further employing ViviRen (R) as the luminescent substrate for the calcium sensitive protein.
The blood-derived sample added in the step (2) is prepared by adding an aqueous PEG solution to the blood of a test subject and collecting a precipitated fraction. For example, 30% PEG6000 is used as the aqueous PEG solution. Moreover, in some cases, a sample derived from the blood of a Graves' disease patient as a positive control and/or a blood sample derived from a normal individual as a negative control can also be used and each added to the cell in the step (2), respectively. The incubation time in the step (2) is not limited and can be set to 30-60 min, for example, 30 min. As a result, cAMP is accumulated in the cell. Thus, more stable, strong luminescence than that in the absence of culture after sample addition can be measured immediately after addition of the CaCl₂ solution.
As long as the incubation times in the steps (2) and (3) are within approximately 4 hours in total, it is not required to perform sterile culture because of the short time.
In the step (2), an anti-TSH antibody may be further added to the cultured cell. The anti-TSH antibody may be a polyclonal antibody or may be a monoclonal antibody. The anti-TSH antibody can be an antibody derived from an appropriate mammal and includes a mouse anti-TSH antibody, a rat anti-TSH antibody, a rabbit anti-TSH antibody and a goat anti-TSH antibody. The anti-TSH antibody may be modified appropriately by those skilled in the art. Moreover, TSH that serves as an antigen is also appropriately selected. TSH that serves as an antigen includes human TSH, mouse TSH, rat TSH, rabbit TSH, feline TSH and canine TSH. Examples of the anti-TSH antibody used in the method of the present invention include a goat anti-human TSH polyclonal antibody.
The CaCl₂-containing solution used in the step (3) may further contain a cation that can be substituted for calcium (e.g., a cadmium ion or a strontium ion), a magnesium ion, a zinc ion, a sulfuric acid ion and/or a carbonic acid ion. The concentrations of Ca²⁺, the cation that can be substituted for calcium, the magnesium ion, the zinc ion, the sulfuric acid ion and the carbonic acid ion that may be contained in the CaCl₂ solution can be set appropriately by those skilled in the art such that the cell can be maintained and the calcium sensitive protein such as aequorin appropriately emits luminescence.
In the step (3), the luminescence is emitted by the calcium sensitive protein such as aequorin immediately after addition of the CaCl₂ solution and can be measured by a method which is well known by those skilled in the art. For example, a luminometer capable of automatically and continuously performing stirring and measurement (PerkinElmer Inc., ARVO-Sx) is used, and the amount of luminescence can be measured by integrating luminescence values for 15-30 s after stirring. The apparatus that can be used in the measurement of the amount of luminescence can be selected appropriately by those skilled in the art.

As a result of carrying out the method, when the amount of luminescence of the calcium sensitive protein (e.g., aequorin) emitted from the cell treated with the sample derived from the blood of a test subject is higher than that emitted from the cell treated with a sample derived from the blood of a normal individual, the test subject can be diagnosed as Graves' disease. Moreover, the serum concentration of an antibody can be determined and compared with a standard antibody concentration of a Graves' disease patient and/or a normal individual to diagnose the test subject as Graves' disease or not.

For example, the amount of luminescence emitted from the cell treated with a blood sample of a test subject and the amount of luminescence emitted from the cell treated with the same amount of a blood sample (standard) of a normal individual as that of the blood sample of the test subject are measured. When the amount of luminescence emitted from the cell treated with the blood sample of the test subject is higher than that emitted from the cell treated with the blood sample (standard) of a normal individual, the serum concentration of a thyroid stimulating antibody (TSAb) of the test subject can be determined to be higher than that of the normal individual. Thus, the test subject can be diagnosed as Graves' disease.
Moreover, the respective amounts of luminescence (integrated values) from the cells treated with blood samples of a large population of normal individuals, for example, 50 to 100 normal individuals, are measured in advance, and a mean thereof and standard deviation (SD) may be calculated. When the amount of luminescence emitted from the cell treated with a blood sample of a test subject is higher than the mean + nSD (e.g., n = 1, 2, 3, 4, or 5) of the amounts of luminescence (integrated values) emitted from the cells treated with the blood samples of the normal individual population, the serum concentration of a thyroid stimulating antibody of the test subject can be determined to be higher than that of the normal individuals. Thus, the test subject can be diagnosed as Graves' disease.

Furthermore, the amount of luminescence (integrated value) emitted from the cell treated with a blood sample of a test subject / the amount of luminescence (integrated value) emitted from the cell treated with a blood sample of a normal individual × 100 (%) may be calculated (this calculated value is defined as a calculated value D). The respective amounts of luminescence (integrated values) emitted from the cells treated with respective blood samples of a large population of normal individuals and a large population of untreated Graves' disease patients (e.g., 50 to 100 individuals per population) are measured in advance, and a cutoff value is set such that a true positive rate of Graves' disease and/or a true negative rate (i.e., being a normal individual) are each, for example, 80% or more, 90% or more, 92% or more, 94% or more, 96% or more, or 98% or more. When the calculated value D is higher than the cutoff value, the test subject can also be diagnosed as Graves' disease. The cutoff value can be set appropriately depending on the properties of a target population, such as age and sex and can be set to, for example, 120%, 130%, 140%, 150%, 160%, 170%, 180%, 190%, or 200%.
Furthermore, an antibody (TSAb) standard (e.g., NIBSC 90/672 or 65/122) is also used, and its concentration may be serially diluted to prepare a calibration curve. With reference to the calibration curve, the actual serum concentration of an antibody (TSAb) is calculated from the measured amount of luminescence of the calcium sensitive protein in the cell treated with a blood sample of a test subject. This concentration can be compared with the previously measured serum concentration of an antibody (TSAb) of each of a large population of normal individuals and a large population of untreated Graves' disease patients (e.g., 50 to 100 individuals per population), or known data reported in a document to diagnose the test subject as Graves' disease or not. For example, when the concentration is higher than the mean +
nSD (e.g., n = 1, 2, 3, 4, or 5) of the serum concentrations of (TSAb) of the normal individual population, the test subject can also be diagnosed as Graves' disease.

Moreover, as a result of carrying out the method, when the amount of luminescence of the calcium sensitive protein (e.g., aequorin) emitted from the cell treated with the sample derived from the blood of a test subject is higher than that emitted from the cell treated with a sample derived from the blood of a normal individual and is lower than that emitted from the cell treated with a sample derived from the blood of a Graves' disease patient, the test subject can be determined as a human having a high risk of developing Graves' disease. Alternatively, the serum concentration of an antibody can be determined and compared with a standard antibody concentration of a Graves' disease patient and/or a normal individual to diagnose the test subject as a human having a high risk of developing Graves' disease or not.
Furthermore, in the step (2), samples taken before and after the treatment of the same Graves' disease patient are added as the sample derived from the blood of a test subject. The amount of luminescence of the calcium sensitive protein (e.g., aequorin) emitted from the cell can be compared between the samples to determine the effectiveness of the treatment. As a result of the test, when the amount of luminescence of the calcium sensitive protein such as aequorin is lower in the sample taken after the treatment than in the sample taken before the treatment, the treatment can be determined as being effective.

The present invention also provides a method for diagnosing hypothyroidism, comprising the following steps (A) to (C):
(A) preparing a mixture containing a cell as defined above, a luminescent substrate for the calcium sensitive protein, a Ca²⁺-free medium, TSH or a stimulating TSAb monoclonal antibody and a sample derived from the blood of a test subject;
(B) adding a Ca²⁺-containing solution to the mixture prepared in (A); and
(C) quantifying the luminescence of the calcium sensitive protein emitted from the cell.
The Ca²⁺-free medium used in the step (A) of the method can be selected appropriately by those skilled in the art and can be a Ca²⁺/Mg²⁺-free medium.
The Ca²⁺-containing solution used in the step (B) of the method can be selected appropriately by those skilled in the art and can be, for example, a CaCl₂ solution. Moreover, the Ca²⁺-containing solution may further contain a cation that can be substituted for calcium (e.g., a cadmium ion or a strontium ion), a magnesium ion, a zinc ion, a sulfuric acid ion, and/or a carbonic acid ion.
The order of addition of the substances described in the step (A) of the method can be set appropriately by those skilled in the art.
For example, a method for diagnosing hypothyroidism (e.g., Hashimoto's disease) is provided, which comprises the following steps:
(1) culturing a cell expressing a thyroid stimulating hormone receptor (TSHR), a cAMP dependent calcium channel and a calcium sensitive protein, in a Ca²⁺-free medium supplemented with a luminescent substrate for the calcium sensitive protein;
(2) adding a sample derived from the blood of a test subject together with TSH to the cultured cell, which is further cultured; and
(3) adding a CaCl₂ solution to the cultured cell, and measuring the luminescence of the calcium sensitive protein emitted from the cell.
The steps (1)-(3) can be carried out appropriately by those skilled in the art with reference to the method for diagnosing Graves' disease described above.
In some cases, in the step (2), a sample derived from the blood of a normal individual as a negative control and/or a sample derived from the blood of a hypothyroidism patient as a positive control can be used, and each of them can be added to the cell, respectively. Moreover, the incubation time in the step (2) is not limited and can be set to 30-120 min, for example, 30 min. Furthermore, in the step (2), TSH can be bovine TSH. In some cases, TSAb can be added instead of or in addition to TSH. TSAb can be a monoclonal antibody.

As a result of carrying out the method, when the amount of luminescence of the calcium sensitive protein (e.g., aequorin) emitted from the cell treated with the sample derived from the blood of a test subject is lower than that emitted from the cell treated with a sample derived from the blood of a normal individual, the test subject can be diagnosed as hypothyroidism. Moreover, the serum concentration of an antibody can be determined and compared with a standard antibody concentration of a hypothyroidism patient and/or a normal individual to diagnose the test subject as hypothyroidism or not. For example, the amount of luminescence emitted from the cell treated with a blood sample of a test subject and the amount of luminescence emitted from the cell treated with the same amount of a blood sample (standard) of a normal individual as that of the blood sample of the test subject are measured. When the amount of luminescence emitted from the cell treated with the blood sample of a test subject is lower than that emitted from the cell treated with the blood sample (standard) of a normal individual, the serum concentration of a thyroid stimulation blocking antibody (TSBAb) of the test subject can be determined to be higher than that of the normal individual. Thus, the test subject can be diagnosed as hypothyroidism.
Moreover, the respective amounts of TSH-induced luminescence (integrated values) of the calcium sensitive proteins in the cells treated with blood samples of a population of a large number of normal individuals, for example, 50 to 100 normal individuals, are measured in advance, and a mean thereof and standard deviation (SD) may be calculated. When the amount of TSH-induced luminescence from the cell treated with a blood sample of a test subject is lower than the mean - nSD (e.g., n = 1, 2, 3, 4, or 5) of the amounts of TSH-induced luminescence (integrated values) from the cells treated with the blood samples of the normal individual population, the serum concentration of a thyroid stimulation blocking antibody (TSBAb) of the test subject can be determined to be higher than that of the normal individuals. Thus, the test subject can also be diagnosed as hypothyroidism.

Furthermore, the amount of luminescence (integrated value) in the cell treated with a blood sample of a test subject / the amount of luminescence (integrated value) from the cell treated with a blood sample of a normal individual × 100 (%) may be calculated (hereinafter, this calculated value is defined as a calculated value E). The respective amounts of luminescence (integrated values) attributed to respective blood samples of a large population of normal individuals and a large population of untreated hypothyroidism patients (e.g., 50 to 100 individuals per population) are measured in advance, and a cutoff value is set such that a true positive rate of hypothyroidism and/or a true negative rate (i.e., being a normal individual) are each, for example, 80% or more, 90% or more, 92% or more, 94% or more, 96% or more, or 98% or more. When the calculated value E is lower than the cutoff value, the test subject can also be diagnosed as hypothyroidism. The cutoff value can be set appropriately depending on the properties of a target population, such as age and sex and can be set to, for example, 10%, 20%, 30%, 40%, 50%, 60%, 70%, 80%, or 90%.
Furthermore, an antibody (TSBAb) standard having a known concentration is also used, and its concentration may be serially diluted to prepare a calibration curve. With reference to the calibration curve, the actual serum concentration of an antibody (TSBAb) is calculated from the measured amount of luminescence emitted from the cell treated with a blood sample of a test subject. This concentration can be compared with the previously measured serum concentration of an antibody (TSBAb) of each of a large population of normal individuals and a large population of untreated hypothyroidism patients (e.g., 50 to 100 individuals per population), or known data reported in a document to diagnose the test subject as hypothyroidism or not. For example, when the concentration is higher than the mean + nSD (e.g., n = 1, 2, 3, 4, or 5) of the serum concentrations of (TSBAb) in the normal individual population, the test subject can also be diagnosed as hypothyroidism.
Moreover, as a result of carrying out the method, when the amount of luminescence of the calcium sensitive protein (e.g., aequorin) emitted from the cell treated with the sample derived from the blood of a test subject is lower than that emitted from the cell treated with a sample derived from the blood of a normal individual and is higher than that emitted from the cell treated with a sample derived from the blood of a hypothyroidism patient, the test subject can be determined as a human having a high risk of developing hypothyroidism. Alternatively, the serum concentration of an antibody can be determined and compared with a standard antibody concentration in a hypothyroidism patient and/or a normal individual to determine the test subject as a human having a high risk of developing hypothyroidism or not. Furthermore, in the step (2), samples taken before and after the treatment of the same hypothyroidism patient are added as the sample derived from the blood of a test subject. The amount of luminescence of the calcium sensitive protein (e.g., aequorin) emitted from the cell can be compared between the samples to determine the effectiveness of the treatment. As a result of the test, when the amount of luminescence of the calcium sensitive protein such as aequorin is higher in sample taken after the treatment than in the sample taken before the treatment, the treatment can be determined as being effective.

Further provided is a method for diagnosing hypothyroidism, comprising the following steps (A) to (C):
(A) preparing a mixture containing a cell according to any of claims 1 to 10, a luminescent substrate for the calcium sensitive protein, a Ca²⁺-containing medium, TSH or a stimulating TSAb monoclonal antibody and a sample derived from the blood of a test subject;
(B) adding forskolin to the mixture prepared in (A); and
(C) quantifying the luminescence of the calcium sensitive protein emitted from the cell.
The Ca²⁺-containing medium used in the step (A) of the method can be selected appropriately by those skilled in the art and can be, for example, a medium containing CaCl₂. Moreover, the Ca²⁺-containing medium may further contain a cation that can be substituted for calcium (e.g., a cadmium ion or a strontium ion), a magnesium ion, a zinc ion, a sulfuric acid ion, and/or a carbonic acid ion. The concentrations of Ca²⁺, the cation that can be substituted for calcium, the magnesium ion, the zinc ion, the sulfuric acid ion and the carbonic acid ion that may be contained in the Ca²⁺-containing medium can be set appropriately by those skilled in the art such that the cell can be maintained and the calcium sensitive protein such as aequorin appropriately emits luminescence.
The order of addition of substances described in the step (A) of the method can be set appropriately by those skilled in the art. For example, a method for diagnosing hypothyroidism (e.g., Hashimoto's disease) is provided, which comprises the following steps:
(1) culturing a cell expressing a thyroid stimulating hormone receptor (TSHR), a cAMP dependent calcium channel and a calcium sensitive protein, in a Ca²⁺-containing medium treated with a luminescent substrate for the calcium sensitive protein;
(2) adding a sample derived from the blood of a test subject together with TSH to the cultured cell, which is further cultured; and
(3) adding forskolin to the cultured cell, and measuring the luminescence of the calcium sensitive protein emitted from the cell.
The steps (1)-(3) can be carried out appropriately by those skilled in the art with reference to the method for diagnosing Graves' disease described above.
In some cases, in the step (2), a sample derived from the blood of a normal individual as a negative control and/or a sample derived from the blood of a hypothyroidism patient as a positive control can be used and each of them can be added to the cell, respectively. Moreover, the incubation time in the step (2) is not limited and can be set to 10-120 min, for example, 10 min. Furthermore, in the step (2), TSH can be bovine TSH. In some cases, TSAb can be added instead of or in addition to TSH. TSAb can be a monoclonal antibody.
In the step (3), the concentration of forskolin can be set appropriately by those skilled in the art.

As a result of carrying out the method, when the amount of luminescence of the calcium sensitive protein (e.g., aequorin) emitted from the cell treated with the sample derived from the blood of a test subject is higher than that emitted from the cell treated with a sample derived from the blood of a normal individual, the test subject can be diagnosed as hypothyroidism. Moreover, the serum concentration of an antibody can be determined and compared with a standard antibody concentration in a hypothyroidism patient and/or a normal individual to diagnose the test subject as hypothyroidism or not. For example, the amount of luminescence emitted from the cell treated with a blood sample of a test subject and the amount of luminescence emitted from the cell treated with the same amount of a blood sample (standard) of a normal individual as that of the blood sample of the test subject are measured. When the amount of luminescence emitted from the cell treated with the blood sample of a test subject is higher than that emitted from the cell treated with the blood sample (standard) of a normal individual, the serum concentration of a thyroid stimulation blocking antibody (TSBAb) of the test subject can be determined to be higher than that of the normal individual. Thus, the test subject can be diagnosed as hypothyroidism.
Moreover, the respective amounts of luminescence (integrated values) from the cells treated with blood samples of a population of a large number of normal individuals, for example, 50 to 100 normal individuals, are measured in advance, and a mean thereof and standard deviation (SD) may be calculated. When the amount of luminescence emitted from the cell treated with a blood sample of a test subject is higher than the mean + nSD (e.g., n = 1, 2, 3, 4, or 5) of the amounts of luminescence (integrated values) emitted from the cells treated with the blood samples of the normal individual population, the serum concentration of a thyroid stimulation blocking antibody (TSBAb) of the test subject can be determined to be higher than that of the normal individuals. Thus, the test subject can also be diagnosed as hypothyroidism.

Furthermore, the amount of luminescence (integrated value) emitted from the cell treated with a blood sample of a test subject / the amount of luminescence (integrated value) emitted from the cell treated with a blood sample of a normal individual × 100 (%) may be calculated (hereinafter, this calculated value is defined as a calculated value F). The respective amounts of luminescence (integrated values) attributed to respective blood samples of a large population of normal individuals and a large population of untreated hypothyroidism patients (e.g., 50 to 100 individuals per population) are measured in advance, and a cutoff value is set such that a true positive rate of hypothyroidism and/or a true negative rate (i.e., being a normal individual) are each, for example, 80% or more, 90% or more, 92% or more, 94% or more, 96% or more, or 98% or more. When the calculated value F is higher than the cutoff value, the test subject can also be diagnosed as hypothyroidism. The cutoff value can be set appropriately depending on the properties of a target population, such as age and sex and can be set to, for example, 150%, 200%, 300%, 400%, 500%, 600%, 700%, 800%, or 900%.
Furthermore, an antibody (TSBAb) standard having a known concentration is also used, and its concentration may be serially diluted to prepare a calibration curve. With reference to the calibration curve, the actual serum concentration of an antibody (TSBAb) is calculated from the measured amount of luminescence emitted from the cell treated with a blood sample of a test subject. This concentration can be compared with the previously measured serum concentration of an antibody (TSBAb) of each of a large population of normal individuals and a large population of untreated hypothyroidism patients (e.g., 50 to 100 individuals per population), or known data reported in a document to diagnose the test subject as hypothyroidism or not. For example, when the concentration is higher than the mean + nSD (e.g., n = 1, 2, 3, 4, or 5) of the serum concentrations of (TSBAb) of the normal individual population, the test subject can also be diagnosed as hypothyroidism.

Moreover, as a result of carrying out the method, when the amount of luminescence of the calcium sensitive protein (e.g., aequorin) emitted from the cell treated with the sample derived from the blood of a test subject is higher than that emitted from the cell treated with a sample derived from the blood of a normal individual and is lower than that emitted from the cell treated with a sample derived from the blood of a hypothyroidism patient, the test subject can be determined as a human having a high risk of developing hypothyroidism. Alternatively, the serum concentration of an antibody can be determined and compared with a standard antibody concentration of a hypothyroidism patient and/or a normal individual to determine the test subject as a human having a high risk of developing hypothyroidism or not.
Furthermore, in the step (2), samples taken before and after the treatment of the same hypothyroidism patient are added as the sample derived from the blood of a test subject. The amount of luminescence of the calcium sensitive protein (e.g., aequorin) emitted from the cell can be compared between the samples to determine the effectiveness of the treatment. As a result of the test, when the amount of luminescence of the calcium sensitive protein such as aequorin is lower in the sample taken after treatment than in the sample taken before the treatment, the treatment can be determined as being effective.

Hereinafter, the present invention will be further described with reference to Examples.

### Examples

### 1. Details on method for constructing frozen cell

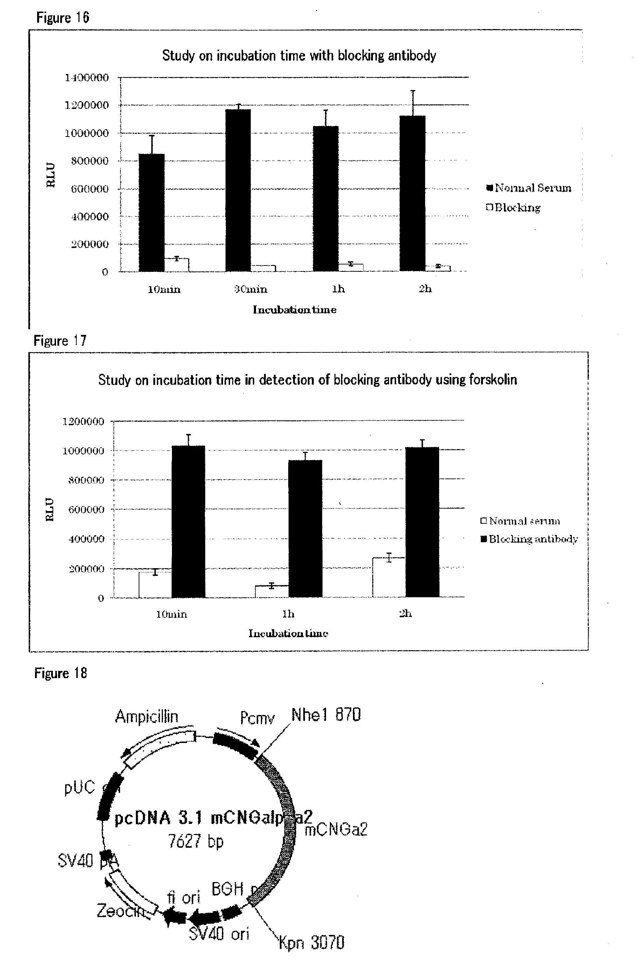

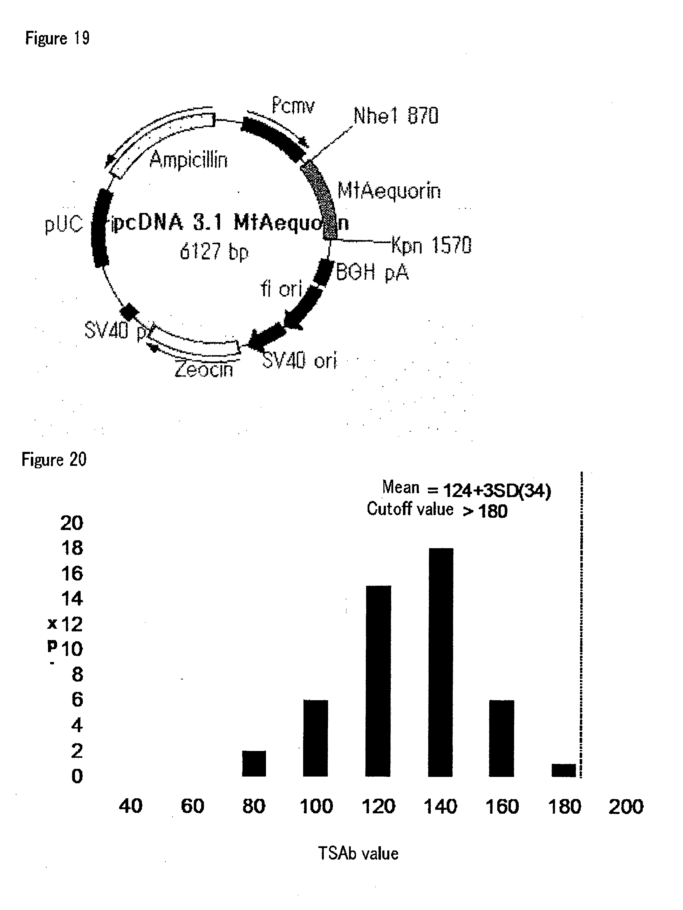

The CDNA sequence of a human thyroid stimulating hormone receptor (Genbank No. NM_000369) (SEQ ID NO: 5) was amplified by the PCR method from a human thyroid gland-derived cDNA library and cloned into pUC18. The hTSHR cDNA cloned in pUC18 was cleaved with BamHI and recloned into a pZeoSV2 vector (Invitrogen Corp.) to prepare pZeoSV2 hTSHR.
The cDNA sequence of a cyclic nucleotide dependent calcium channel (Genbank No. BC048775) (SEQ ID NO: 4) was amplified by the PCR method from a mouse olfactory epithelial cell-derived cDNA library and cloned into a 1994-bp expression vector (pCMVSPORT, Invitrogen Corp.) to prepare pmCNGa2 (Figure 18). Furthermore, for enhancing cAMP selectivity and sensitivity thereto, a construct pmCNGα2MW expressing a modified cyclic nucleotide dependent calcium channel (SEQ ID NO: 6) in which 460th cysteine (C) is substituted with tryptophan (W) and the 583rd glutamic acid (E) is substituted with methionine (M) was prepared by the point-mutation PCR method. Moreover, a synthetic apoaequorin cDNA sequence (676 bp) (SEQ ID NO: 7) that was optimized for human codon usage by the oligo DNA elongation method, and that has a mitochondrial targeting signal was treated with restriction enzymes KpnI and NheI and cloned into pcDNA3.1 (Invitrogen Corp.) treated with KpnI and NheI to prepare an apoaequorin expression vector pcDNA mt sAEQ (Figure 19). CHO cells were seeded at a cell concentration of 1.0×10⁵ cells/ml into a 10-cm² Petri dish. On the next day, the cells were transfected with 1 µg of pZeoSV2 hTSHR, 2 µg of pmCNGα2MW, and 2 µg of pcDNA mt sAEQ per Petri dish using FuGENE6 (TM) (Roche Applied Science). On the next day, the cells were dissociated from the Petri dish by the addition of 400 µl of a Versene solution (EDTA) to the Petri dish and suspended in a DMEM/F12 medium containing 10 ml of 5% cFCS. The obtained suspension was centrifuged at 1000 rpm for 5 min. Then, the pellet was dissolved at a concentration of 2-5 × 10⁶ cells/ml in 1 ml of CELLBANKER and stored at -80°C.

### 2. Concentration dependent curve and lowest detection sensitivity of bovine TSH (bTSH) obtained using frozen cell

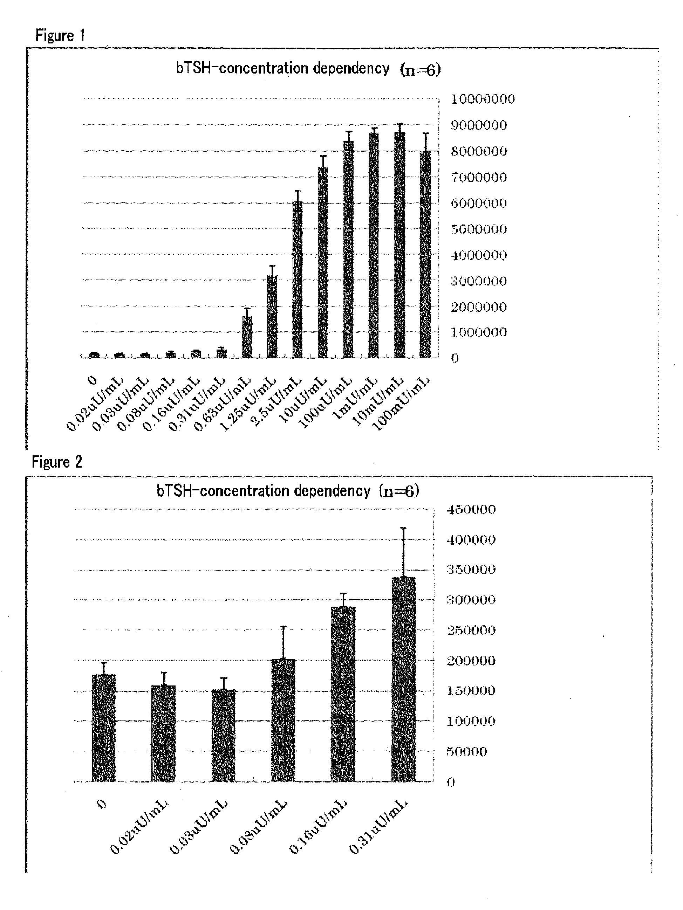

1 ml (3 × 10⁶ cells/ml) of the frozen cells prepared in 1 was thawed in a warm bath and suspended in 10 ml of a sample buffer (130 mM NaCl, 5 mM KCl, 20 mM HEPES, 1 mM MgCl₂, 4.8 mM NaHCO₃, 5% PEG6000, pH 7.4). After centrifugation at 1000 rpm for 5 min, 10 ml of a sample buffer was added to the obtained precipitate, and 7.5 µl of 4 mM ViviRen (Promega Corp.) was added thereto. The mixture was seeded at a concentration of 90 µl/well into a 96-well plate. After culture at 37°C for 3 hours in a CO₂ atmosphere, bTSH serially diluted with PBS was added thereto (n = 6) at a concentration of 10 µl/well, and the cells were cultured for 30 min. Then, 3 mM CaCl₂ solution was added thereto at a concentration of 50 µl/well, and the amount of luminescence was measured using a luminometer (PerkinElmer Inc., ARVO-Sx; hereinafter, the same model was used in Examples). A bTSH concentration dependent curve is shown in Figure 1.

As shown in Figure 2 (magnified view of the low concentration range), the minimum detectable quantity for bTSH was 0.16 µU/ml so that the detected value was able to be significantly discriminated from the value which is calculated by blank value + 3SD. Moreover, since aequorin performs luminescent reaction, a high signal to blank ratio (S/N ratio) (i.e., a 100 µU/ml of bTSH, S/N ratio of approximately 45 times (9000000/200000)) was obtained by the method of the present invention.

As shown in Figure 2 (magnified view of the low concentration range), the minimum detectable quantity for bTSH was 0.16 µU/ml so that the detected value was able to be significantly discriminated from the value which is calculated by blank value + 3SD. Thus, the sensitivity was an order of magnitude higher than that of the existing kit available from YAMASA CORP: minimum detectable quantity for bTSH, 1 µU/mL (Atsuo Nagata, et al.: CLINICAL ENDOCRINOLOGY, 41: 1023, 1993). Moreover, since aequorin performs luminescent reaction, the high signal to blank ratio (S/N ratio), (i.e., a 100 µU/ml of bTSH, S/N ratio of approximately 45 times (9000000/200000)), obtained by the method of the present invention was far superior to that of the YAMASA kit (100 µU/ml of bTSH, SN = 7).

### 3. Study on reproducibility

A human Graves' disease patient-derived TSAb standard: MRC Research standard B 1966 Long-acting Thyroid Stimulator (NIBSC: National Institute for Biological Standards and Control, code 65/122) was diluted with a normal individual serum to prepare H (1.88 mU LAST/ml), M (1.25 mU LAST/ml), and L (0.94 mU LAST/ml) control samples. 150 µL of 30% PEG6000 was added to 50 µl of each prepared control serum, and the mixtures were stirred and then left standing at 4°C for 5 min. After centrifugation at 3000 rpm at 4°C for 20 min, the obtained precipitates were separately dissolved in 400 µl of a sample buffer to prepare sample solutions. 1 ml (3 × 10⁶ cells/ml) of the frozen cells prepared in 1 was thawed in a warm bath and suspended in 10 ml of a sample buffer. After centrifugation at 1000 rpm for 5 min, 10 ml of a sample buffer was added to the obtained precipitate, and 7.5 µl of 4 mM ViviRen was added thereto. The mixture was seeded at a concentration of 80 µl/well into a 96-well plate. After culture at 37°C for 3 h in a CO₂ atmosphere, the prepared samples were separately added thereto (n = 2) at a concentration of 20 µl/well, and the cells were cultured for 30 min. Then, 9 mM CaCl₂ solution was added thereto at a concentration of 50 µl/well, and the amount of luminescence was measured using a luminometer. Comparison was made in the case where each six of the L, M, and H samples were simultaneously purified and assayed (within-run reproducibility), the case where the L, M, and H samples were independently purified and assayed at different 10 days (between-run reproducibility), and the case where the L, M, and H samples of different production lots were purified and assayed on the same day (lot-to-lot reproducibility). In addition to the assay samples, H (576 TSAb%), M (342 TSAb%), and L (197 TSAb%) separately scored using the YAMASA kit were added to the plate, and a calibration curve was drawn. A value determined from the obtained regression line was defined as a sample concentration (TSAb% based on the YAMASA kit) (YAMASA TSAb% = Value of sample/Value of normal individual serum × 100 (%)).

**[Table 1]**

| | Within-run reproducibility | | | Between-run reproducibility | | | Lot-to-lot reproducibility | | |
|---|---|---|---|---|---|---|---|---|---|
| n | 6 | | | 10 | | | 3 | | |
| | L | M | H | L | M | H | L | M | H |
| Maximum value | 242 | 387 | 587 | 248 | 333 | 583 | 190 | 343 | 543 |
| Minimum value | 186 | 293 | 471 | 188 | 280 | 470 | 171 | 307 | 492 |
| Mean | 217 | 364 | 525 | 202 | 309 | 512 | 183 | 322 | 512 |
| SD | 20 | 37 | 40 | 18 | 15 | 36 | 11 | 19 | 27 |
| CV (%) | 9 | 10 | 8 | 9 | 5 | 7 | 6 | 6 | 5 |

The method of the present invention was confirmed to produce high reproducibility because the assay system had high sensitivity and a high S/N ratio. Moreover, 5-6% CV demonstrated that the method of the present invention had high lot-to-lot reproducibility. The assay system with little difference between different lots could be constructed using the CHO cells and the optimized transfection conditions.
The CV value of the reproducibility of the existing TSAb kit available from YAMASA CORP. has been reported to be 10-15%, demonstrating high reproducibility of the method of the present invention.
Moreover, since the existing kit is produced by freezing porcine tissue-derived thyroid gland cells, its problem is that lot-to-lot variation is large due to individual difference among pigs from which the tissue is derived (lot-to-lot CV: 10-19 %). By contrast, the method of the present invention was confirmed to have high reproducibility even among different lots.

### 4. Study on incubation time after addition of substrate (ViviRen) for aequorin luminescence

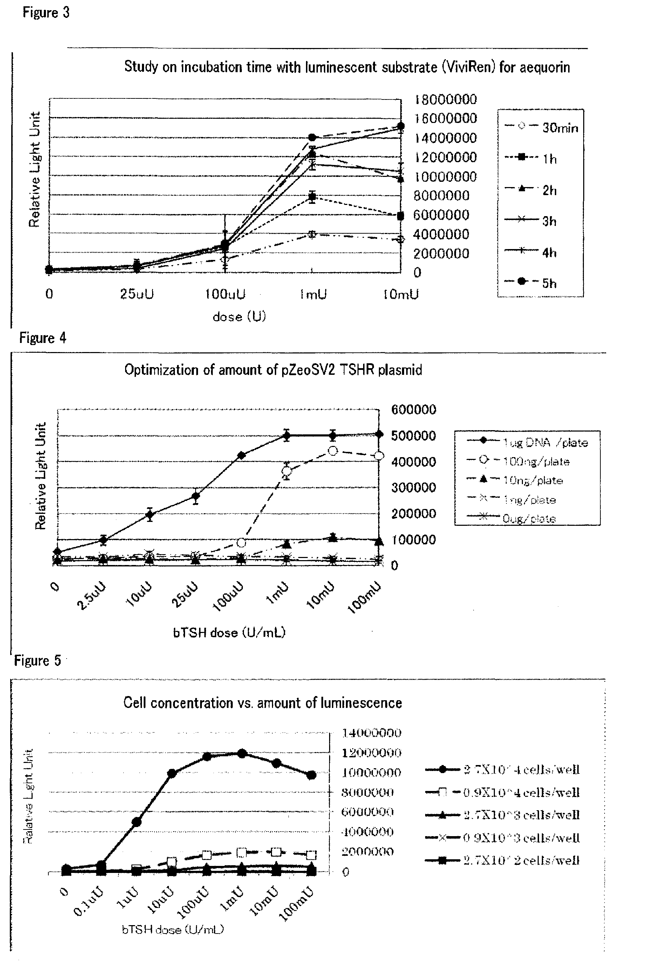

1 ml (3 × 10⁶ cells/ml) of the frozen cells prepared in 1 was thawed in a warm bath and suspended in 10 ml of a sample buffer. After centrifugation at 1000 rpm for 5 min, 10 ml of a sample buffer was added to the obtained precipitate, and 7.5 µl of 4 mM ViviRen was added thereto. After the addition, 30 min, or 1, 2, 3, 4, 5, 6, 7, or 8 h, the mixture was seeded at a concentration of 90 µl/well into a 96-well plate. bTSH serially diluted with PBS was added thereto at a concentration of 10 µl/well, and the cells were cultured for 30 min. Then, 9 mM CaCl₂ solution was added thereto at a concentration of 50 µl/well, and the amount of luminescence was measured using a luminometer.
It was demonstrated that the amount of luminescence reached a plateau at 2 hours after ViviRen addition (see Figure 3).

### 5. Study on amount of receptor plasmid used in transfection

10 ml of CHO cells having a concentration of 1 × 10⁵ cells/ml was seeded into a 10-cm² Petri dish and cultured for 1 day. Then, 3 ug of pcDNA mt sAEQ, 1 ug of pmCNGα2MW, and 0 to 1 ug of pZeoSV2 TSHR plasmid were mixed and further mixed with 600 µl of DMEM/F12 and 18 µl of FuGENE6 (Roche Applied Science), and transfection was performed. After further overnight culture, the medium was removed, and the cells were washed with 10 ml of PBS. Then, 800 µl of Versene was added thereto, and the cells were cultured at 37°C for 5 min and then suspended in 10 ml of DMEM/F12 cFCS. After centrifugation at 1000 rpm for 5 min, the precipitate was dissolved in 10 ml of DMEM/F12 cFCS, and 6.5 µl of 4 mM ViviRen was added thereto. After culture at 37°C for 3 h, the medium was replaced by PBS, and the culture solution was seeded at a concentration of 90 µl/well to a 96-well plate. 30 min after addition of a concentration series of bTSH, 3 mM CaCl₂ solution was added thereto at a concentration of 50 µl/well, and the amount of luminescence was measured using a luminometer.
It was demonstrated that the receptor plasmid in an amount of 1 ug/plate in transfection was required for obtaining the optimum amount of luminescence (see Figure 4).

### 6. Optimization of cell concentration

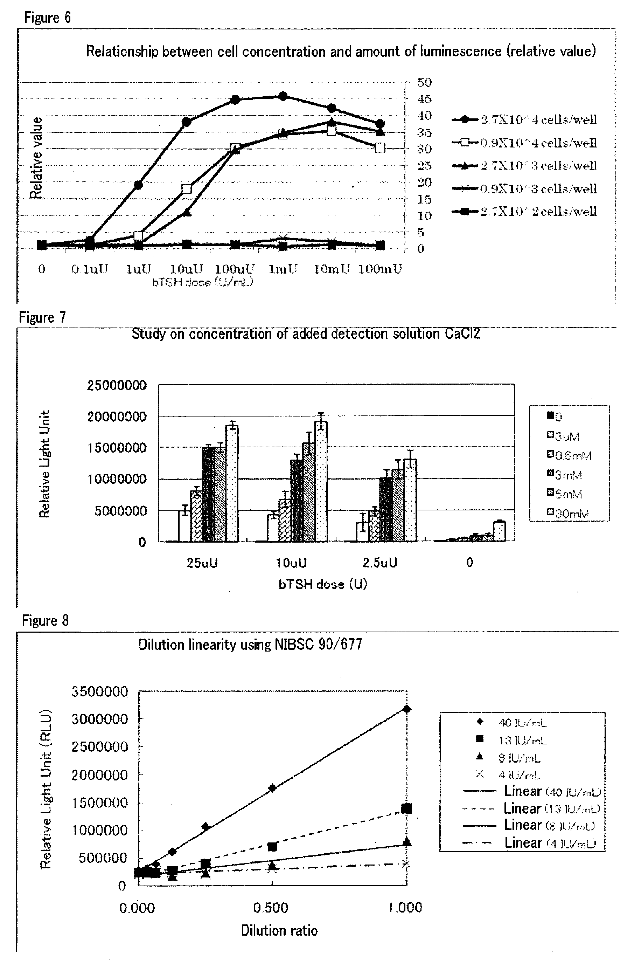

1 ml (3 × 10⁶ cells/ml) of the frozen cells prepared in 1 was thawed in a warm bath and suspended in 10 ml of a sample buffer. After centrifugation at 1000 rpm for 5 min, 10 ml of a sample buffer was added to the obtained precipitate. A cell series whose cell concentration was adjusted to concentrations 3 × 10³ cells/ml to 3 × 10⁵ cells/ml was prepared. 7.5 µl of 4 mM ViviRen was added thereto, and the mixture was seeded at a concentration of 90 µl/well into a 96-well plate. After culture at 37°C for 3 h in a CO₂ atmosphere, bTSH serially diluted with PBS was added thereto at a concentration of 10 µl/well, and the cells were cultured for 30 min. Then, 9 mM CaCl₂ solution was added thereto at a concentration of 50 µl/well, and the amount of luminescence was measured using a luminometer.
As shown in Figures 5 and 6, the amount of luminescence was increased in a cell concentration dependent manner. It was demonstrated that 3 × 10⁵ cells/ml was the optimum concentration, based on the relative value where each blank value was defined as 1.

### 7. Study on concentration of added CaCl₂ detection solution

1 ml (3 × 10⁶ cells/ml) of the frozen cells prepared in 1 was thawed in a warm bath and suspended in 10 ml of a sample buffer. After centrifugation at 1000 rpm for 5 min, 10 ml of a sample buffer was added to the obtained precipitate, and 7.5 µl of 4 mM ViviRen was added thereto. The mixture was seeded at a concentration of 90 µL/well into a 96-well plate. After culture at 37°C for 3 h in a CO₂ atmosphere, bTSH serially diluted with PBS was added thereto at a concentration of 10 µL/well, and the cells were cultured for 30 min. Then, a CaCl₂ ·detection solution (final concentrations: 0 to 30 mM) was added thereto at a concentration of 50 µl/well, and the amount of luminescence was measured using a luminometer.
It was demonstrated that the optimum CaCl₂ concentrations were 3 to 6 mM at which a low value at background (0) and the high amount of luminescence in the sample were obtained (see Figure 7).

### 8. Study on dilution linearity using stimulating antibody control serum

1 ml (3 × 10⁶ cells/ml) of the frozen cells prepared in 1 was thawed in a warm bath and suspended in 10 mof a sample buffer. After centrifugation at 1000 rpm for 5 min, 10 ml of a sample buffer was added to the obtained precipitate, and 7.5 µl of 4 mM ViviRen was added thereto. The mixture was seeded at a concentration of 80 µl/well into a 96-well plate. The cells were cultured at 37°C for 3 h in a CO₂ atmosphere. IgG fractions of a positive control serum series (adhering to Roche NIBSC 90/672; 40 IU/ml, 13 IU/ml, 8 IU/ml, and 4 IU/ml) purified and diluted with 30% PEG6000 were separately diluted 1/2, 1/4, 1/8, 1/16, or 1/32-fold with a sample buffer, and each solution was added thereto at a concentration of 20 µl/well. After culture for 30 min, 9 mM CaCl₂ solution was added thereto at a concentration of 50 µl/well, and the amount of luminescence was measured using a luminometer.
As shown in Figure 8, it was confirmed that the amount of luminescence was increased with linearity.

### 9. Study on method for detecting thyroid stimulation blocking antibody (TSBAb)

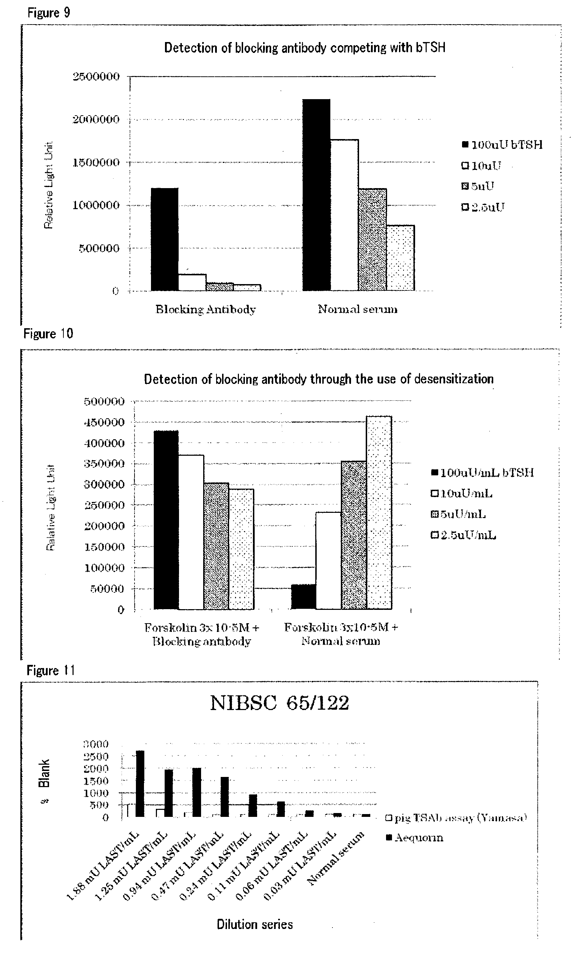

1 ml (3 × 10⁶ cells/ml) of the frozen cells prepared in 1 was thawed in a warm bath and suspended in 10 m of a sample buffer. After centrifugation at 1000 rpm for 5 min, 10 ml of a sample buffer was added to the obtained precipitate, and 7.5 µl of 4 mM ViviRen was added thereto. The mixture was seeded at a concentration of 90 µ/well into a 96-well plate. After culture at 37°C for 3 h in a CO₂ atmosphere, a hypothyroidism patient-derived thyroid stimulation blocking antibody (TSBAb) fraction and a normal individual serum-derived IgG fraction purified with 30% PEG6000 were separately added thereto at a concentration of 10 µl/well. At the same time, bTSH (final concentrations: 100 µU to 2.5 µU/ml) was further added thereto at a concentration of 10 µl/well. After culture for 30 min, 9 mM CaCl₂ solution was added thereto at a concentration of 50 µL/well, and the amount of luminescence was measured using a luminometer.
It was demonstrated that 90% or more luminescence was inhibited in the hypothyroidism patient-derived antibody fraction in the presence of 2.5 to 10 µU/ml bTSH (see Figure 9). Approximately 50% inhibition was seen in the presence of 100 µU/mL bTSH, demonstrating competitive inhibition according to the concentration of competing bTSH (see Figure 9).
When the blocking antibody is detected, reduction in signal in the presence of bTSH is generally observed. Conventional methods are known to have the disadvantage that the S/N ratio is as low as 5 to 10-fold and a measurement range becomes narrow due to the low S/N ratio. By contrast, the method of the present invention has a wide measurement range such that the S/N ratio in the presence or absence of bTSH is 50-fold. Thus, the blocking antibody can be detected with high sensitivity.

### 10. Study on method for detecting thyroid stimulation blocking antibody (TSBAb) by cell desensitization

1 ml (3 × 10⁶ cells/ml) of the frozen cells prepared in 1 was thawed in a warm bath and suspended in 10 ml of a sample buffer. After centrifugation at 1000 rpm for 5 min, 10 ml of a DMEM/F12 cFCS medium containing calcium was added to the obtained precipitate, and 7.5 µl of 4 mM ViviRen was added thereto. The mixture was seeded at a concentration of 90 µl/well into a 96-well plate. After culture at 37°C for 3 h in a CO₂ atmosphere, a hypothyroidism patient-derived thyroid stimulation blocking antibody (TSBAb) fraction and a normal individual serum-derived IgG fraction purified with 30% PEG6000 were separately added thereto at a concentration of 10 µl/well. At the same time, bTSH (final concentrations: 100 µU to 2.5 µU/ml) was further added at a concentration of 10 µl/well. After culture for 30 min, 10⁻⁴ M forskolin solution was added thereto at a concentration of 50 µl/well, and the amount of luminescence was measured using a luminometer.
In Figure 10, no difference between the blocking antibody and normal individual samples was seen due to insufficient desensitization in the presence of bTSH with concentrations of 5 µU or lower. However, in the presence of 100 µU bTSH, the amount of luminescence was decreased in the normal individual serum, and the increased amount of luminescence was observed in the presence of the blocking antibody, demonstrating that according to the method of the present invention, the presence or absence of the blocking antibody can be detected using desensitization.

### [Mechanism of luminescence in the presence or absence of blocking antibody according to the method of the present invention]

Under this condition, calcium is present in a medium at the time of bTSH addition to cells; thus luminescent reaction occurs immediately after bTSH addition in the absence of the blocking antibody as seen in normal individual sera. By contrast, in the presence of the blocking antibody (TSBAb), TSBAb inhibits the action of bTSH, so that luminescent reaction does not occur. In this state, upon subsequent addition of forskolin, which activates adenylate cyclase, cAMP is formed to cause luminescence without the mediation of a TSH receptor. However, in the absence of TSBAb, the cells are already desensitized by luminescent reaction that has occurred once via adenylate cyclase activated by bTSH. Thus, luminescence does not occur even by the further addition of forskolin as the second stimulus. By contrast, in the presence of TSBAb, the blocking antibody prevents bTSH from activating adenylate cyclase. Thus, upon subsequent addition of forskolin, cAMP is formed to cause luminescent reaction.

### [Discussion]

Conventional methods for detecting a blocking antibody are indicated by % inhibition with respect to a normal individual. However, the problem of indication using % inhibition is a lack of quantitative reliability in a high concentration range of 90% or more.
The method of the present invention using cell desensitization action and forskolin can be indicated by increase in the amount of luminescence with respect to a normal individual. Thus, the upper limit is not defined, and the presence or absence of the blocking antibody can be detected with linearity even in a high concentration range.

### 11. Sensitivity comparison with existing kit using stimulating antibody standard

1 ml (3 × 10⁶ cells/ml) of the frozen cells prepared in 1 was thawed in a warm bath and suspended in 10 ml of a sample buffer. After centrifugation at 1000 rpm for 5 min, 10 ml of a sample buffer was added to the obtained precipitate, and 7.5 µl of 4 mM ViviRen was added thereto. The mixture was seeded at a concentration of 90 µl/well into a 96-well plate. MRC Research standard B 1966 (NIBSC 65/122) was diluted with a normal individual serum to prepare a concentration series. 150 µl of 30% PEG6000 was added to 50 µl of each control sample in the prepared series, and the mixtures were stirred and then left standing at 4°C for 5 min. After centrifugation at 3000 rpm at 4°C for 20 min, the obtained precipitates were separately dissolved in 50 µl of a sample buffer to prepare sample solutions. After culture at 37°C for 3 h in a CO₂ atmosphere, the sample solutions were separately added thereto at a concentration of 10 µl/well, and the cells were cultured for 30 min. 9 mM CaCl₂ detection solution was added thereto at a concentration of 50 µl/well, and the amount of luminescence was measured using a luminometer.
Values separately measured using the existing YAMASA kit using the porcine thyroid gland are also shown. The existing YAMASA kit resulted in 200 TSAb% at 0.94 mU LAST/ml and failed to detect concentrations below the sensitivity threshold. By contrast, the method of the present invention was confirmed to be able to detect the stimulating antibody with 2018 TSAB% at 0.94 mU LAST/ml and with 242 TSAb% even at 0.06 mU LAST/ml and to be able to detect the stimulating antibody with great sensitivity which is about 16 times higher than that of the existing kit (see Figure 11).

### 12. Study on time-dependent change in luminescence using kit using frozen cell

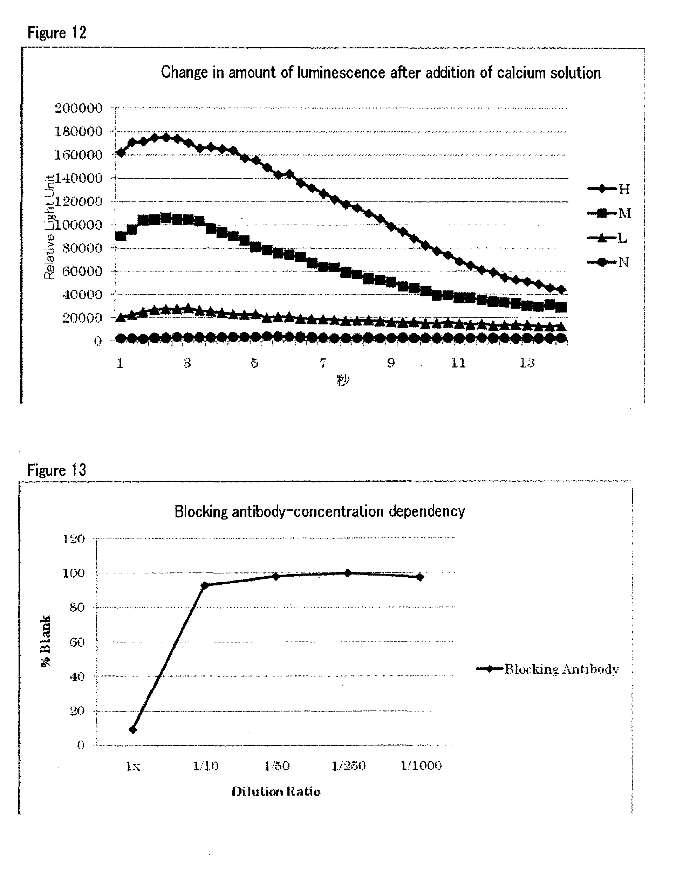

1 ml (3 × 10⁶ cells/ml) of the frozen cells prepared in 1 was thawed in a warm bath and suspended in 10 ml of a sample buffer (130 mM NaCl, 5 mM KCl, 20 mM HEPES, 1 mM MgCl₂, 4.8 mM NaHCO₃, 5% PEG6000, pH 7.4). After centrifugation at 1000 rpm for 5 min, 10 ml of a sample buffer was added to the obtained precipitate, and 7.5 µl of 4 mM ViviRen (Promega Corp.) was added thereto. The mixture was seeded at a concentration of 90 µl/well into a 96-well plate. After culture at 37°C for 3 h in a CO₂ atmosphere, H (high value), M (median value), and L (low value) samples purified with 30% PEG6000 were separately added thereto (n = 6) at a concentration of 10 µl/well. After culture for 30 min, 9 mM CaCl₂ solution was added thereto at a concentration of 50 µl/well and stirred for 3 s. Then, the amount of luminescence was measured over times for 13 s using a luminometer. As a result, as shown in Figure 12, relatively stable luminescence was observed as soon as the CaCl₂ solution was added.

### 13. Study on quantitative reliability of thyroid stimulation blocking antibody (TSBAb)

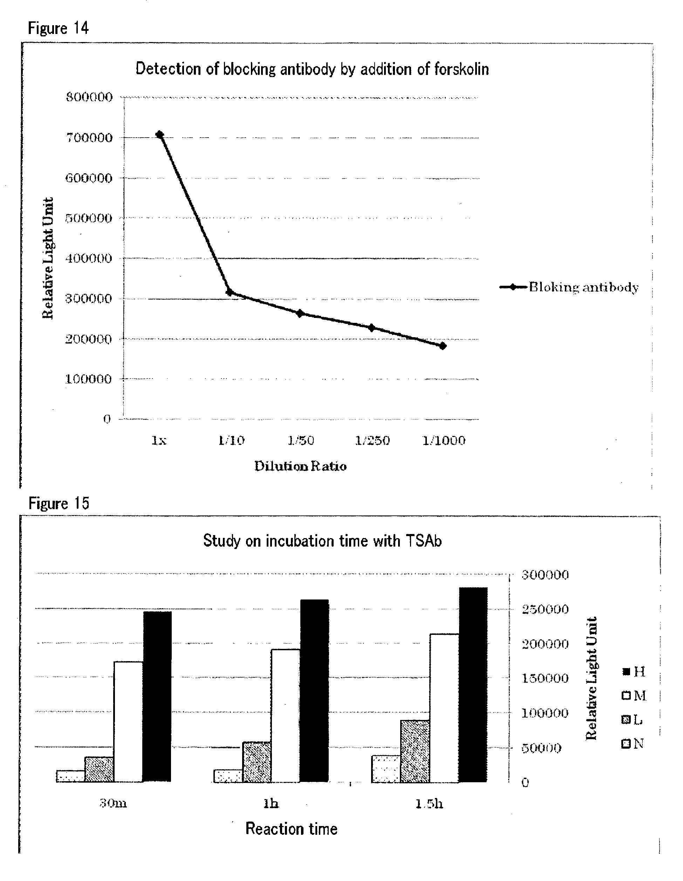

(1) Method for detecting thyroid stimulation blocking antibody (TSBAb) using competitive inhibition against bTSH 1 ml (3 × 10⁶ cells/ml) of the frozen cells prepared in 1 was thawed in a warm bath and suspended in 10 ml of a sample buffer (130 mM NaCl, 5 mM KCl, 20 mM HEPES, 1 mM MgCl₂, 4.8 mM NaHCO₃, 5% PEG6000, pH 7.4). After centrifugation at 1000 rpm for 5 min, 10 ml of a sample buffer was added to the obtained precipitate, and 7.5 µl of 4 mM ViviRen (Promega Corp.) was added thereto. After culture at 37°C for 3 h in a CO₂ atmosphere, 90 µl of the cell solution was mixed with 10 µl of each sample in a dilution series of a hypothyroidism patient-derived blocking antibody purified with 30% PEG6000, and cultured for 30 min. Then, 9 mM CaCl₂ solution was added thereto at a concentration of 50 µl/well and the amount of luminescence was measured using a luminometer. As shown in Figure 13, the thyroid stimulation blocking antibody (TSBAb) was quantitatively detected using this frozen cell.
(2) Concentration dependence of method for detecting thyroid stimulation blocking antibody (TSBAb) using cell desensitization 1 ml (3 × 10⁶ cells/ml) of the frozen cells prepared in 1 was thawed in a warm bath and suspended in 10 ml of a sample buffer (3 mM CaCl₂, 130 mM NaCl, 5 mM KCl, 20 mM HEPES, 1 mM MgCl₂, 4.8 mM NaHCO₃, 5% PEG6000, pH 7.4). After centrifugation at 1000 rpm for 5 min, 10 ml of a sample buffer was added to the obtained precipitate, and 7.5 µl of 4 mM ViviRen (Promega Corp.) was added thereto. The mixture was seeded at a concentration of 90 µl/well into a 96-well plate. Each sample in a dilution series of a hypothyroidism patient-derived blocking antibody purified with 30% PEG6000 was added thereto at a concentration of 10 µl/well, and further, a bTSH solution (final concentration: 100 µl/ml) was added thereto at a concentration of 10 µl/well. After culture at 37°C for 30 min in a CO₂ atmosphere, 10⁻⁴ M forskolin solution was added thereto at a concentration of 50 µL/well, and the amount of luminescence was measured using a luminometer.
As shown in Figure 14, the thyroid stimulation blocking antibody (TSBAb) was detected in a concentration dependent manner using this frozen cell.

### 14. Study on duration of action (incubation time) of thyroid stimulating antibody (TSAb) or thyroid stimulation blocking antibody (TSBAb)

(1) Method for detecting stimulating antibody (TSAb) 1 ml (3 × 10⁶ cells/ml) of the frozen cells prepared in 1 was thawed in a warm bath and suspended in 10 ml of a sample buffer (130 mM NaCl, 5 mM KCl, 20 mM HEPES, 1 mM MgCl₂, 4.8 mM NaHCO₃, 5% PEG6000, pH 7.4). After centrifugation at 1000 rpm for 5 min, 10 ml of a sample buffer was added to the obtained precipitate, and 7.5 µl of 4 mM ViviRen (Promega Corp.) was added thereto.
   The mixture was seeded at a concentration of 90 µL/well into a 96-well plate. High value (H), median value (M), low value (L), and normal individual (N) serum samples purified with 30% PEG6000 were separately added thereto at a concentration of 10 µl/well. After culture for 30 min to 1.5 h, 9 mM CaCl₂ solution was added thereto at a concentration of 50 µL/well, and the amount of luminescence was measured using a luminometer.
   As shown in Figure 15, the amount of luminescence reached a plateau after 30-min to 1-h duration of action of the stimulating antibody (TSAb) on the cellaccording to the present invention.
(2) Study on duration of action in method for detecting thyroid stimulation blocking antibody (TSBAb) using competitive inhibition against bTSH
   1 ml (3 × 10⁶ cells/ml) of the frozen cells prepared in 1 was thawed in a warm bath and suspended in 10 ml of a sample buffer (130 mM NaCl, 5 mM KCl, 20 mM HEPES, 1 mM MgCl₂, 4.8 mM NaHCO₃, 5% PEG6000, pH 7.4). After centrifugation at 1000 rpm for 5 min, 10 ml of a sample buffer was added to the obtained precipitate, and 7.5 µl of 4 mM ViviRen (Promega Corp.) was added thereto. The mixture was seeded at a concentration of 90 µl/well into a 96-well plate, and the cells were cultured for 3 h. A hypothyroidism patient-derived serum sample and a normal individual serum sample purified with 30% PEG6000 were separately added thereto at a concentration of 10 µl/well, and further, a bTSH solution (final concentration: 10 µU/mL) was added thereto at a concentration of 10 µl/well. After culture for 10 min to 2 h, 9 mM CaCl₂ solution was added thereto at a concentration of 50 µl/well, and the amount of luminescence was measured using a luminometer.
   As shown in Figure 16, the amount of luminescence reached a plateau after 30-min duration of action of bTSH (competing with the blocking antibody (TSBAb)) on the cell according to the present invention.
(3) Method for detecting thyroid stimulation blocking antibody (TSBAb) using cell desensitization
   1 ml (3 × 10⁶ cells/ml) of the frozen cells prepared in 1 was thawed in a warm bath and suspended in 10 ml of a CO₂ independent medium (Cat No. 18045, Invitrogen Corp.). After centrifugation at 1000 rpm for 5 min, 10 ml of a CO₂ independent medium was added to the obtained precipitate, and 7.5 µl of 4 mM ViviRen (Promega Corp.) was added thereto. The mixture was seeded at a concentration of 90 µl/well into a 96-well plate, and the cells were cultured for 3 h. A hypothyroidism patient-derived serum sample and a normal individual serum sample purified with 30% PEG6000 were separately added thereto at a concentration of 10 µl/well, and further, a bTSH solution (final concentration: 100 µU/ml) was added thereto at a concentration of 10 µl/well. After culture for 10 min to 2 h, 10⁻⁴ M forskolin solution was added thereto at a concentration of 50 µl/well, and the amount of luminescence was measured using a luminometer.
   As shown in Figure 17, the amount of luminescence reached a plateau after 10-min duration of action of bTSH (competing with the blocking antibody (TSBAb)) on the cell according to the present invention.

### 15. Assay using various thyroid disease patient samples

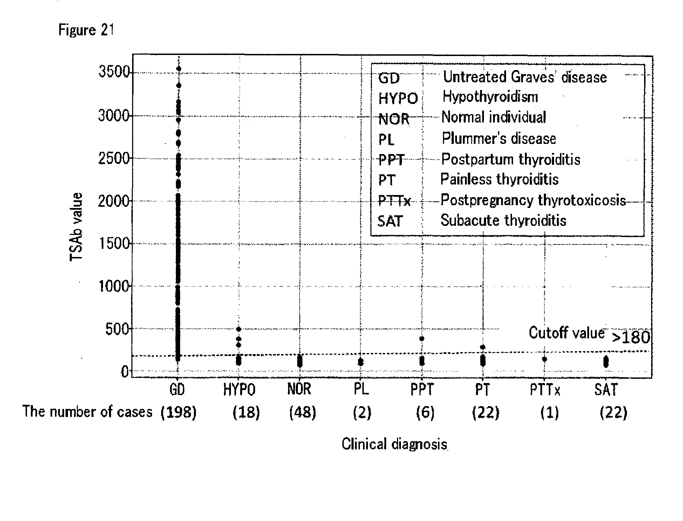

The frozen cells prepared in 1 and a diagnostic kit for Graves' disease provided by the present invention containing a medium for cell culture, a cell washing solution, ViviRen, a calcium solution, a goat anti-hTSH antibody, a control serum and a 96-well plate described below were used to study serum TSAb activity in various thyroid disease patient groups. Specifically, the sera of 198 untreated Graves' disease patients, 18 hypothyroidism patients, 48 normal individuals, 2 Plummer's disease cases, 6 postpartum thyroiditis cases, 22 painless thyroiditis cases and 22 subacute thyroiditis cases, all of which were clinically and definitely diagnosed, were used (see Table 2). 150 µl of 30% PEG6000 was added to 50 µl of the serum of each patient, and the mixtures were stirred and then left standing at 4°C for 5 min. After centrifugation at 3000 rpm at 4°C for 20 min, the obtained precipitates were separately dissolved in 400 µl of a medium for cell culture (5% PEG6000, CO₂ independent medium (calcium chloride, D-calcium pantothenate, L-glutamine, phenol red-free, Invitrogen Corp.)) to prepare sample solutions. The sample solutions were separately added (n = 2) at a concentration of 10 µl/well to a 96-well plate. 1 ml (3 × 10⁶ cells/ml) of the frozen cells was thawed in a warm bath and suspended in 10 ml of a cell washing solution (CO₂ independent medium). After centrifugation at 1000 rpm for 5 min, 12 ml of a medium for cell culture was added to the obtained precipitate to prepare a cell solution. 7.5 µl of 4 mM ViviRen was added to the cell solution, and the mixture was seeded at a concentration of 90 µL/well into a 96-well plate. After culture for 4 h, 3 mM calcium solution was added thereto at a concentration of 100 µl/well, and the amount of luminescence was measured using a luminometer. Among hypothyroidism patients, there is a patient having a high hTSH value in serum. Thus, for neutralizing the action of hTSH, 1.2 µl of a goat anti-hTSH polyclonal antibody (Meridian Life Science, Inc.) was added to the medium for cell culture, and assay was performed under this condition. A measured value in each patient was calculated based on a one-point calibration curve obtained by defining, as 1800, the measured value of a control serum containing NIBSC 65/122 diluted (1.874 mU LAST/ml) with a normal individual serum. Moreover, the sera of the same patients as above were assayed using the existing kit (TSAb kit, YAMASA CORP.) according to the instruction included in the kit.
A TSAb cutoff value was set from the serum TSAb values of normal individuals. The frequency distribution of serum TSAb values of 48 normal individuals is shown in Figure 20. The mean of the values in the normal individuals was 124 ± 34, showing normal distribution. The cutoff value was set to mean + 3SD = 180.
The TSAb value of each thyroid disease is shown in Figure 21. 195 out of 198 untreated Graves' disease cases showed positive (98%). 3 hypothyroidism cases showed positive, and each one of painless thyroiditis and postpartum thyroiditis cases showed positive. In table 3, a true positive ratio of Graves' disease was studied using 198 untreated Graves' disease patients in the existing kit and the kit of the present invention. The true positive ratio of the existing kit was 68%, and that of the kit provided by the present invention was 98%, demonstrating that the kit of the present invention was far superior in Graves' disease detection sensitivity to the existing kit.

**[Table 2]**

| Disease | n | Existing kit | | | Kit provided by the present invention | | |
|---|---|---|---|---|---|---|---|
| | | Positive | Negative | True positive ratio | Positive | Negative | True positive ratio |
| Untreated Graves' disease(GD) | 198 | 134 | 64 | 68 | 195 | 3 | 98 |
| Hypothyroidism (Hypo) | 18 | 3 | 15 | | 3 | 15 | |
| Normal individual (Nomal) | 48 | 0 | 48 | | 0 | 48 | |
| Plummer's disease (PL) | 2 | 0 | 2 | | 0 | 2 | |
| Postpartum thyroiditis (PPT) | 6 | 0 | 6 | | 1 | 5 | |
| Painless thyroiditis (PT) | 22 | 0 | 22 | | 1 | 21 | |
| Subacute thyroiditis (SAT) | 22 | 0 | 22 | | 0 | 22 | |

**[Table 3]**

| | | Clinical-definite diagnosis | | | | | Clinical-definite diagnosis | | |
|---|---|---|---|---|---|---|---|---|---|
| | | Posi tive | Nega tive | Total | | | Posi tive | Nega tive | Total |
| Existing kit (porcine TSAb) | Positive | 134 | 3 | 137 | Kit provided by the present invention | Positive | 195 | 5 | 200 |
| | Negative | 64 | 115 | 179 | | Negative | 3 | 113 | 116 |
| | Total | 198 | 118 | 316 | | Total | 198 | 118 | 316 |
| True positive ratio | 68% | | | | True positive ratio | 98% | | | |
| True negative ratio | 97% | | | | True negative ratio | 96% | | | |
| Concorda nce rate | 79% | | | | Concordance rate | 97% | | | |

### Industrial Applicability

The present invention can provide a method and a kit for determining a TSH receptor antibody, which are easy to manipulate and are safe. Thus, thyroid disease can be diagnosed.

## Claims

1. A kit for diagnosing thyroid disease, comprising a cell expressing a thyroid stimulating hormone receptor (TSHR), a cAMP dependent calcium channel and a calcium sensitive protein, wherein the calcium sensitive protein is a protein that emits luminescence in response to calcium.

2. The kit according to claim 1, further comprising an anti-TSH antibody.

3. The kit according to claim 1 or 2, further comprising ViviRen (R) as a luminescent substrate for the calcium sensitive protein.

4. The kit according to any one of claims 1-3, further comprising TSH or a stimulating TSAb monoclonal antibody.

5. The kit according to claim 4, further comprising forskolin.

6. The kit according to any one of claims 1-5, wherein the TSHR is TSHR having the amino acid sequence represented by SEQ ID NO:1, the cAMP dependent calcium channel is a modified CNG calcium channel having the amino acid sequence represented by SEQ ID NO:2 and the calcium sensitive protein is modified apoaequorin having the amino acid sequence represented by SEQ ID NO:3.

7. The kit according to any one of claims 1-6, wherein the cell is selected from the group consisting of a CHO cell, a HEK293 cell and a 3T3 cell.

8. A kit according to any one of claims 1-7 for use in diagnosing thyroid disease.

9. The kit of claim 8, wherein the amount of a thyroid stimulating antibody and/or the amount of a thyroid stimulation blocking antibody in a biological sample, is determined.

10. The kit according to claim 8 or 9, for diagnosing Graves' disease and/or Hashimoto's disease.

11. A method for diagnosing thyroid disease, for determining a human having a high risk of developing thyroid disease, or for determining the effectiveness of treatment of thyroid disease, comprising the following steps (1), (2), and (3) or (1'), (2), and (3):
(1) preparing a mixture containing the cell comprised in the kit according to any one of claims 1-7, a luminescent substrate for the calcium sensitive protein, a Ca²⁺-free medium or a Ca²⁺/Mg²⁺-free medium, TSH and a sample derived from the blood of a test subject; or
(1') preparing a mixture containing the cell comprised in the kit according to any one of claims 1-7, a luminescent substrate for the calcium sensitive protein, a Ca²⁺-free medium or a Ca²⁺/Mg²⁺-free medium and a sample derived from the blood of a test subject;
(2) adding a Ca²⁺-containing solution to the mixture prepared in (1) or (1'); and
(3) measuring the luminescence of the calcium sensitive protein emitted from the cell.

12. The method according to claim 11, wherein the mixture prepared in (1') further contains an anti-TSH antibody.

13. A method for diagnosing hypothyroidism, comprising the following steps (1) to (3):
(1) preparing a mixture containing the cell comprised in the kit according to any one of claims 1-7, a luminescent substrate for the calcium sensitive protein, a Ca²⁺-containing medium, TSH or a stimulating TSAb monoclonal antibody and a sample derived from the blood of a test subject;
(2) adding forskolin to the mixture prepared in (1); and
(3) quantifying the luminescence of the calcium sensitive protein emitted from the cell.

14. The method according to any one of claims 11-13, wherein the cell is not cultured in a sterile environment.

15. The method according to any one of claims11-14, wherein the TSHR is TSHR having the amino acid sequence represented by SEQ ID NO:1, the cAMP dependent calcium channel is a modified CNG calcium channel having the amino acid sequence represented by SEQ ID NO:2, the calcium sensitive protein is modified apoaequorin having the amino acid sequence represented by SEQ ID NO:3 and the luminescent substrate for the calcium sensitive protein is ViviRen (R).

## Patentansprüche

1. Kit zum Diagnostizieren einer Schilddrüsenerkrankung, umfassend eine Zelle, die einen Thyreotropinrezeptor (TSHR), einen cAMP-abhängigen Calciumkanal und ein calciumempfindliches Protein exprimiert, wobei das calciumempfindliche Protein ein Protein ist, das als Reaktion auf Calcium eine Lumineszenz emittiert.

2. Kit gemäß Anspruch 1, der weiterhin einen Anti-TSH-Antikörper umfasst.

3. Kit gemäß Anspruch 1 oder 2, der weiterhin ViviRen (R) als lumineszierendes Substrat für das calciumempfindliche Protein umfasst.

4. Kit gemäß einem der Ansprüche 1 bis 3, der weiterhin TSH oder einen stimulierenden monoklonalen TSAb-Antikörper umfasst.

5. Kit gemäß Anspruch 4, der weiterhin Forskolin umfasst.

6. Kit gemäß einem der Ansprüche 1 bis 5, wobei der TSHR ein TSHR ist, der die Aminosäuresequenz aufweist, die durch SEQ ID Nr. 1 dargestellt wird, der cAMP-abhängige Calciumkanal ein modifizierter CNG-Calciumkanal ist, der die Aminosäuresequenz aufweist, die durch SEQ ID Nr. 2 dargestellt wird, und das calciumempfindliche Protein modifiziertes Apoaequorin ist, das die Aminosäuresequenz aufweist, die durch SEQ ID Nr. 3 dargestellt wird.

7. Kit gemäß einem der Ansprüche 1 bis 6, wobei die Zelle aus der Gruppe ausgewählt ist, die aus einer CHO-Zelle, einer HEK293-Zelle und einer 3T3-Zelle besteht.

8. Kit gemäß einem der Ansprüche 1 bis 7 zur Verwendung beim Diagnostizieren einer Schilddrüsenerkrankung.

9. Kit gemäß Anspruch 8, wobei die Menge eines schilddrüsenstimulierenden Antikörpers und/oder die Menge eines die Schilddrüsenstimulation blockierenden Antikörpers in einer biologischen Probe bestimmt wird.

10. Kit gemäß Anspruch 8 oder 9 zum Diagnostizieren von Morbus Basedow und/oder Hashimoto-Thyreoiditis.

11. Verfahren zum Diagnostizieren einer Schilddrüsenerkrankung zur Bestimmung eines Menschen, der ein hohes Risiko aufweist, eine Schilddrüsenerkrankung zu entwickeln, oder zur Bestimmung der Wirksamkeit einer Behandlung einer Schilddrüsenerkrankung, umfassend die folgenden Schritte (1), (2) und (3) oder (1'), (2) und (3):
(1) Herstellen eines Gemischs, das die Zelle, die in dem Kit gemäß einem der Ansprüche 1 bis 7 enthalten ist, ein lumineszierendes Substrat für das calciumempfindliche Protein, ein Ca²⁺-freies Medium oder ein Ca²⁺/Mg²⁺-freies Medium, TSH und eine vom Blut eines Probanden stammende Probe enthält; oder
(1') Herstellen eines Gemischs, das die Zelle, die in dem Kit gemäß einem der Ansprüche 1 bis 7 enthalten ist, ein lumineszierendes Substrat für das calciumempfindliche Protein, ein Ca²⁺-freies Medium oder ein Ca²⁺/Mg²⁺-freies Medium und eine vom Blut eines Probanden stammende Probe enthält;
(2) Hinzufügen einer Ca²⁺-haltigen Lösung zu dem in (1) oder (1') hergestellten Gemisch; und
(3) Messen der von der Zelle emittierten Lumineszenz des calcium-empfindlichen Proteins.

12. Verfahren gemäß Anspruch 11, wobei das in (1') hergestellte Gemisch weiterhin einen Anti-TSH-Antikörper enthält.

13. Verfahren zum Diagnostizieren einer Hypothyreose, umfassend die folgenden Schritte (1) bis (3):
(1) Herstellen eines Gemischs, das die Zelle, die in dem Kit gemäß einem der Ansprüche 1 bis 7 enthalten ist, ein lumineszierendes Substrat für das calciumempfindliche Protein, ein Ca²⁺-haltiges Medium, TSH oder einen stimulierenden monoklonalen TSAb-Antikörper und eine vom Blut eines Probanden stammende Probe enthält;
(2) Hinzufügen von Forskolin zu dem in (1) hergestellten Gemisch; und
(3) Quantifizieren der von der Zelle emittierten Lumineszenz des calciumempfindlichen Proteins.

14. Verfahren gemäß einem der Ansprüche 11 bis 13, wobei die Zelle nicht in einer sterilen Umgebung kultiviert wird.

15. Verfahren gemäß einem der Ansprüche 11 bis 14, wobei der TSHR ein TSHR ist, der die Aminosäuresequenz aufweist, die durch SEQ ID Nr. 1 dargestellt wird, der cAMP-abhängige Calciumkanal ein modifizierter CNG-Calciumkanal ist, der die Aminosäuresequenz aufweist, die durch SEQ ID Nr. 2 dargestellt wird, das calciumempfindliche Protein modifiziertes Apoaequorin ist, das die Aminosäuresequenz aufweist, die durch SEQ ID Nr. 3 dargestellt wird, und es sich bei dem lumineszierenden Substrat für das calciumempfindliche Protein um ViviRen (R) handelt.

## Revendications

1. Kit pour diagnostiquer une maladie de la thyroïde, comprenant une cellule exprimant un récepteur de thyréostimuline (TSHR), un canal calcique dépendant de l'AMPc et une protéine sensible au calcium, où la protéine sensible au calcium est une protéine qui émet une luminescence en réponse au calcium.

2. Kit selon la revendication 1, comprenant en outre un anticorps anti-TSH.

3. Kit selon la revendication 1 ou 2, comprenant en outre ViviRen (R) comme substrat luminescent pour la protéine sensible au calcium.

4. Kit selon l'une quelconque des revendications 1-3, comprenant en outre de la TSH ou un anticorps monoclonal TSAb stimulant.

5. Kit selon la revendication 4, comprenant en outre de la forskoline.

6. Kit selon l'une quelconque des revendications 1-5, où le TSHR est un TSHR ayant la séquence d'aminoacides représentée par SEQ ID NO:1, le canal calcique dépendant de l'AMPc est un canal calcique CNG modifié ayant la séquence d'aminoacides représentée par SEQ ID NO:2 et la protéine sensible au calcium est de l'apoaequorine modifiée ayant la séquence d'aminoacides représentée par SEQ ID NO:3.

7. Kit selon l'une quelconque des revendications 1-6, où la cellule est choisie dans le groupe consistant en une cellule CHO, une cellule HEK293 et une cellule 3T3.

8. Kit selon l'une quelconque des revendications 1-7 destiné à être utilisé dans le diagnostic d'une maladie de la thyroïde.

9. Kit selon la revendication 8, où la quantité d'anticorps stimulant la thyroïde et/ou la quantité d'anticorps bloquant la stimulation de la thyroïde dans un échantillon biologique est déterminée.

10. Kit selon la revendication 8 ou 9, pour diagnostiquer la maladie de Graves et/ou la maladie d'Hashimoto.

11. Procédé pour diagnostiquer une maladie de la thyroïde, pour déterminer un humain ayant un risque important de développer une maladie de la thyroïde, ou pour déterminer l'efficacité d'un traitement d'une maladie de la thyroïde, comprenant les étapes (1), (2) et (3) ou (1'), (2) et (3) suivantes :
(1) préparation d'un mélange contenant la cellule comprise dans le kit selon l'une quelconque des revendications 1-7, un substrat luminescent pour la protéine sensible au calcium, un milieu dépourvu de Ca²⁺ ou un milieu dépourvu de Ca²⁺/Mg²⁺, de la TSH, et un échantillon dérivé du sang d'un sujet test ; ou
(1') préparation d'un mélange contenant la cellule comprise dans le kit selon l'une quelconque des revendications 1-7, un substrat luminescent pour la protéine sensible au calcium, un milieu dépourvu de Ca²⁺ ou un milieu dépourvu de Ca²⁺/Mg²⁺ et un échantillon dérivé du sang d'un sujet test ;
(2) addition d'une solution contenant Ca²⁺ au mélange préparé en (1) ou (1') ; et
(3) mesure de la luminescence de la protéine sensible au calcium émise par la cellule.

12. Procédé selon la revendication 11, où le mélange préparé en (1') contient en outre un anticorps anti-TSH.

13. Procédé pour diagnostiquer un hypothyroïdisme comprenant les étapes (1) à (3) suivantes :
(1) préparation d'un mélange contenant la cellule comprise dans le kit selon l'une quelconque des revendications 1-7, un substrat luminescent pour la protéine sensible au calcium, un milieu contenant Ca²⁺, de la TSH ou un anticorps monoclonal TSAb stimulant et un échantillon dérivé du sang d'un sujet test ;
(2) addition de forskoline au mélange préparé en (1) ; et
(3) quantification de la luminescence de la protéine sensible au calcium émise par la cellule.

14. Procédé selon l'une quelconque des revendications 11-13, où la cellule n'est pas cultivée dans un environnement stérile.

15. Procédé selon l'une quelconque des revendications 11-14, où le TSHR est un TSHR ayant la séquence d'aminoacides représentée par SEQ ID NO:1, le canal calcique dépendant de l'AMPc est un canal calcique CNG modifié ayant la séquence d'aminoacides représentée par SEQ ID NO:2, la protéine sensible au calcium est de l'apoaequorine modifiée ayant la séquence d'aminoacides représentée par SEQ ID NO:3 et le substrat luminescent pour la protéine sensible au calcium est ViviRen (R).


### SEQUENCE LISTING

<110> Otsuka Pharmaceutical Co.,Ltd.
<120> A bioassay method and a kit for measuring an antibody against TSH receptor and a novel recombinant cell therefor
<130> 669763
<150> JP 2009-155183
<151> 2009-06-30
<160> 7
<170> PatentIn version 3.2
<210> 1
   <211> 764
   <212> PRT
   <213> Human
<400> 1
<210> 2
   <211> 664
   <212> PRT
   <213> Artificial
<220>
   <223> modified CNG alpha2
<400> 2
   Met Met Thr Glu Lys Ser Asn Gly Val Lys Ser Ser Pro Ala Asn Asn
   1 5 10 15
   His Asn His His Pro Pro Pro Ser Ile Lys Ala Asn Gly Lys Asp Asp
   20 25 30
   His Arg Ala Gly Ser Arg Pro Gln Ser Val Ala Ala Asp Asp Asp Thr
   35 40 45
   Ser Ser Glu Leu Gln Arg Leu Ala Glu Met Asp Thr Pro Arg Arg Gly
   50 55 60
   Arg Gly Gly Phe Arg Arg Ile Val Arg Leu Val Gly Ile Ile Arg Asp
   65 70 75 80
   Trp Ala Asn Lys Asn Phe Arg Glu Glu Glu Pro Arg Pro Asp Ser Phe
   85 90 95
   Leu Glu Arg Phe Arg Gly Pro Glu Leu Gln Thr Val Thr Thr His Gln
   100 105 110
   Gly Asp Gly Lys Gly Asp Lys Asp Gly Glu Gly Lys Gly Thr Lys Lys
   115 120 125
   Lys Phe Glu Leu Phe Val Leu Asp Pro Ala Gly Asp Trp Tyr Tyr Arg
   130 135 140
   Trp Leu Phe Val Ile Ala Met Pro Val Leu Tyr Asn Trp Cys Leu Leu
   145 150 155 160
   Val Ala Arg Ala Cys Phe Ser Asp Leu Gln Arg Asn Tyr Phe Val Val
   165 170 175
   Trp Leu Val Leu Asp Tyr Phe Ser Asp Thr Val Tyr Ile Ala Asp Leu
   180 185 190
   Ile Ile Arg Leu Arg Thr Gly Phe Leu Glu Gln Gly Leu Leu Val Lys
   195 200 205
   Asp Pro Lys Lys Leu Arg Asp Asn Tyr Ile His Thr Leu Gln Phe Lys
   210 215 220
   Leu Asp Val Ala Ser Ile Ile Pro Thr Asp Leu Ile Tyr Phe Ala Val
   225 230 235 240
   Gly Ile His Ser Pro Glu Val Arg Phe Asn Arg Leu Leu His Phe Ala
   245 250 255
   Arg Met Phe Glu Phe Phe Asp Arg Thr Glu Thr Arg Thr Ser Tyr Pro
   260 265 270
   Asn Ile Phe Arg Ile Ser Asn Leu Val Leu Tyr Ile Leu Val Ile Ile
   275 280 285
   His Trp Asn Ala Cys Ile Tyr Tyr Ala Ile Ser Lys Ser Ile Gly Phe
   290 295 300
   Gly Val Asp Thr Trp Val Tyr Pro Asn Ile Thr Asp Pro Glu Tyr Gly
   305 310 315 320
   Tyr Leu Ala Arg Glu Tyr Ile Tyr Cys Leu Tyr Trp Ser Thr Leu Thr
   325 330 335
   Leu Thr Thr Ile Gly Glu Thr Pro Pro Pro Val Lys Asp Glu Glu Tyr
   340 345 350
   Leu Phe Val Ile Phe Asp Phe Leu Ile Gly Val Leu Ile Phe Ala Thr
   355 360 365
   Ile Val Gly Asn Val Gly Ser Met Ile Ser Asn Met Asn Ala Thr Arg
   370 375 380
   Ala Glu Phe Gln Ala Lys Ile Asp Ala Val Lys His Tyr Met Gln Phe
   385 390 395 400
   Arg Lys Val Ser Lys Asp Met Glu Ala Lys Val Ile Lys Trp Phe Asp
                   405                 410                 415
   Tyr Leu Trp Thr Asn Lys Lys Thr Val Asp Glu Arg Glu Val Leu Lys
               420                 425                 430
   Asn Leu Pro Ala Lys Leu Arg Ala Glu Ile Ala Ile Asn Val His Leu
           435                 440                 445
   Ser Thr Leu Lys Lys Val Arg Ile Phe Gln Asp Trp Glu Ala Gly Leu
       450                 455                 460
   Leu Val Glu Leu Val Leu Lys Leu Arg Pro Gln Val Phe Ser Pro Gly
   465                 470                 475                 480
   Asp Tyr Ile Cys Arg Lys Gly Asp Ile Gly Lys Glu Met Tyr Ile Ile
                   485                 490                 495
   Lys Glu Gly Lys Leu Ala Val Val Ala Asp Asp Gly Val Thr Gln Tyr
               500                 505                 510
   Ala Leu Leu Ser Ala Gly Ser Cys Phe Gly Glu Ile Ser Ile Leu Asn
           515                 520                 525
   Ile Lys Gly Ser Lys Met Gly Asn Arg Arg Thr Ala Asn Ile Arg Ser
       530                 535                 540
   Leu Gly Tyr Ser Asp Leu Phe Cys Leu Ser Lys Asp Asp Leu Met Glu
   545                 550                 555                 560
   Ala Val Thr Glu Tyr Pro Asp Ala Lys Lys Val Leu Glu Glu Arg Gly
                   565                 570                 575
   Arg Glu Ile Leu Met Lys Met Gly Leu Leu Asp Glu Asn Glu Val Ala
               580                 585                 590
   Ala Ser Met Glu Val Asp Val Gln Glu Lys Leu Glu Gln Leu Glu Thr
           595                 600                 605
   Asn Met Glu Thr Leu Tyr Thr Arg Phe Ala Arg Leu Leu Ala Glu Tyr
       610                 615                 620
   Thr Gly Ala Gln Gln Lys Leu Lys Gln Arg Ile Thr Val Leu Glu Thr
   625                 630                 635                 640
   Lys Met Lys Gln Asn His Glu Asp Asp Tyr Leu Ser Asp Gly Ile Asn
                   645                 650                 655
   Thr Pro Glu Pro Ala Val Ala Glu
   660
<210> 3
   <211> 228

   <212> PRT <213> Artificial
<220>
   <223> modified aequorin
<400> 3
   Met Ser Val Leu Thr Pro Leu Leu Leu Arg Gly Leu Thr Gly Ser Ala
   1               5                   10                  15
   Arg Arg Leu Pro Val Pro Arg Ala Lys Ile His Ser Leu Pro Pro Glu
               20                  25                  30
   Gly Lys Leu Gly Ile Met Lys Val Lys Leu Thr Ser Asp Phe Asp Asn
           35                  40                  45
   Pro Lys Trp Ile Gly Arg His Lys His Met Phe Asn Phe Leu Asp Val
       50                  55                  60
   Asn His Asn Gly Arg Ile Ser Leu Asp Glu Met Val Tyr Lys Ala Ser
   65                  70                  75                  80
   Asp Ile Val Ile Asn Asn Leu Gly Ala Thr Pro Glu Gln Ala Lys Arg
                   85                  90                  95
   His Lys Asp Ala Val Glu Ala Phe Phe Gly Gly Ala Gly Met Lys Tyr
               100                 105                 110
   Gly Val Glu Thr Glu Trp Pro Glu Tyr Ile Glu Gly Trp Lys Arg Leu
           115                 120                 125
   Ala Thr Glu Glu Leu Glu Arg Tyr Ser Lys Asn Gln Ile Thr Leu Ile
       130                 135                 140
   Arg Leu Trp Gly Asp Ala Leu Phe Asp Ile Ile Asp Lys Asp Gln Asn
   145                 150                 155                 160
   Gly Ala Ile Thr Leu Asp Glu Trp Lys Ala Tyr Thr Lys Ser Ala Gly
                   165                 170                 175
   Ile Ile Gln Ser Ser Glu Asp Cys Glu Glu Thr Phe Arg Val Cys Asp
               180                 185                 190
   Ile Asp Glu Ser Gly Gln Leu Asp Val Asp Glu Met Thr Arg Gln His
           195                 200                 205
   Leu Gly Phe Trp Tyr Thr Met Asp Pro Ala Cys Glu Lys Leu Tyr Gly
       210                 215                 220
   Gly Ala Val Pro
   225
<210> 4
   <211> 1995
   <212> DNA
   <213> Mus musculus
<400> 4
   atgatgaccg aaaaatccaa cggtgtgaag agctctccag ctaataacca taaccatcat      60
   cctcctcctt ctatcaaggc caatggcaaa gatgaccaca gggcaggtag cagaccacag     120
   tctgtggcag ctgatgatga cacttcctca gaactgcaaa ggttggcaga gatggatact     180
   ccacgcaggg gaaggggtgg cttccgaagg attgtccgcc tggtggggat catcagagac     240
   tgggccaaca aaaatttccg tgaggaggaa ccaagacctg actccttcct agagcgtttc     300
   cgtgggcctg agctccagac tgtgacaacc catcaggggg atggcaaagg cgacaaggac     360
   ggcgagggaa agggcaccaa aaagaaattt gaactgtttg ttttggaccc agctggagac     420
   tggtattacc gttggttgtt tgtcattgcc atgcctgttc tttacaactg gtgtctgttg     480
   gtagccagag cctgcttcag tgatctacag agaaactatt ttgtggtatg gctggtgctg     540
   gattacttct cagacactgt ttatattgca gacctcatca ttcggctgcg cacaggcttc     600
   ctagaacaag ggctcctggt caaagacccc aagaaattgc gagacaacta tatccacact     660
   ttgcagttca aattggatgt ggcttctatc atccccactg acctcattta ttttgctgtg     720
   ggtatccaca gccctgaggt acgctttaat cgtctgttac actttgcccg tatgtttgag     780
   ttctttgacc gcactgagac acgtacaagc taccccaaca tcttccgaat cagcaacctg     840
   gtcctctaca tcttggtcat catccactgg aatgcttgta tttactatgc tatctctaag     900
   tccattggct ttggggttga cacctgggtt taccccaaca ttactgaccc tgaatatggc     960
   tacctggcta gggagtacat ttactgcctt tactggtcca cactgaccct caccaccatt    1020
   ggagagacac caccccctgt aaaggatgag gagtacctat ttgtcatctt tgacttcctg    1080
   attggtgtcc tcatctttgc cactattgtg ggaaatgtgg gctccatgat ctccaacatg    1140
   aatgccacac gagcagagtt ccaggccaag attgatgctg tcaaacacta catgcagttc    1200
   cgaaaggtca gtaaagacat ggaagccaag gtcatcaaat ggtttgacta cttgtggacc    1260
   aataagaaga cagtagatga acgagaagtc ctaaagaacc tgcctgcaaa gcttagggca    1320
   gagatagcca ttaatgttca tttgtccact ctgaagaaag tgcgcatatt ccaggattgt    1380
   gaagctggcc tgctggtgga actggtactg aagcttcgtc ctcaggtctt tagtcctgga    1440
   gattatattt gccgtaaggg ggacattggc aaggaaatgt acatcatcaa ggagggcaaa    1500
   ttggcagtgg tagctgatga tggtgtgact cagtatgcct tgctgtcagc tgggagctgc 1560
   tttggtgaga ttagtatcct taacattaag ggtagcaaaa tgggcaatcg gcgcactgcc 1620
   aatatccgta gtctgggcta ctcagatctc ttctgcttgt ccaaggatga tcttatggaa 1680
   gctgtgactg agtatcctga tgccaagaaa gtcctggaag aacggggtag ggagatcctg 1740
   atgaaggaag gtctactgga tgagaatgaa gtggcagcta gtatggaggt agatgttcag 1800
   gagaagctgg agcagctgga gacaaacatg gagaccttgt acactcgttt tgcccgcctg 1860
   ctggctgagt acactggagc ccagcagaag ctcaaacagc gcatcacagt gctggagacc 1920
   aagatgaaac agaaccatga ggatgattac ctatcagatg ggataaacac ccctgagcca 1980
   gctgttgctg aatag 1995
<210> 5
   <211> 2301
   <212> DNA
   <213> Human
<400> 5
   tggaaaatga ggccggcgga cttgctgcag ctggtgctgc tgctcgacct gcccagggac      60
   ctgggcggaa tggggtgttc gtctccaccc tgcgagtgcc atcaggagga ggacttcaga     120
   gtcacctgca aggatattca acgcatcccc agcttaccgc ccagtacgca gactctgaag     180
   cttattgaga ctcacctgag aactattcca agtcatgcat tttctaatct gcccaatatt     240
   tccagaatct acgtatctat agatgtgact ctgcagcagc tggaatcaca ctccttctac     300
   aatttgagta aagtgactca catagaaatt cggaatacca ggaacttaac ttacatagac     360
   cctgatgccc tcaaagagct cccccctcct aagttccttg gcattttcaa cactggactt     420
   aaaatgttcc ctgacctgac caaagtttat tccactgata tattctttat acttgaaatt     480
   acagacaacc cttacatgac gtcaatccct gtgaatgctt ttcagggact atgcaatgaa     540
   accttgacac tgaagctgta caacaatggc tttacttcag tccaaggata tgctttcaat     600
   gggacaaagc tggatgctgt ttacctaaac aagaataaat acctgacagt tattgacaaa     660
   gatgcatttg gaggagtata cagtggacca agcttgctgg acgtgtctca aaccagtgtc     720
   actgcccttc catccaaagg cctggagcac ctgaaggaac tgatagcaag aaacacctgg     780
   actcttaaga aacttccact ttccttgagt ttccttcacc tcacacgggc tgacctttct     840
   tacccaagcc actgctgtgc ttttaagaat cagaagaaaa tcagaggaat ccttgagtcc     900
   ttgatgtgta atgagagcag tatgcagagc ttgcgccaga gaaaatctgt gaatgccttg     960
   aatagccccc tccaccagga atatgaagag aatctgggtg acagcattgt tgggtacaag    1020
   gaaaagtcca agttccagga tactcataac aacgctcatt attacgtctt ctttgaagaa    1080
   caagaggatg agatcattgg ttttggccag gagctcaaaa acccccagga agagactcta    1140
   caagcttttg acagccatta tgactacacc atatgtgggg acagtgaaga catggtgtgt    1200
   acccccaagt ccgatgagtt caacccgtgt gaagacataa tgggctacaa gttcctgaga 1260
   attgtggtgt ggttcgttag tctgctggct ctcctgggca atgtctttgt cctgcttatt 1320
   ctcctcacca gccactacaa actgaacgtc ccccgctttc tcatgtgcaa cctggccttt 1380
   gcggatttct gcatggggat gtacctgctc ctcatcgcct ctgtagacct ctacactcac 1440
   tctgagtact acaaccatgc catcgactgg cagacaggcc ctgggtgcaa cacggctggt 1500
   ttcttcactg tctttgcaag cgagttatcg gtgtatacgc tgacggtcat caccctggag 1560
   cgctggtatg ccatcacctt cgccatgcgc ctggaccgga agatccgcct caggcacgca 1620
   tgtgccatca tggttggggg ctgggtttgc tgcttccttc tcgccctgct tcctttggtg 1680
   ggaataagta gctatgccaa agtcagtatc tgcctgccca tggacaccga gacccctctt 1740
   gctctggcat atattgtttt tgttctgacg ctcaacatag ttgccttcgt catcgtctgc 1800
   tgctgttatg tgaagatcta catcacagtc cgaaatccgc agtacaaccc aggggacaaa 1860
   gataccaaaa ttgccaagag gatggctgtg ttgatcttca ccgacttcat atgcatggcc 1920
   ccaatctcat tctatgctct gtcagcaatt ctgaacaagc ctctcatcac tgttagcaac 1980
   tccaaaatct tgctggtact cttctatcca cttaactcct gtgccaatcc attcctctat 2040
   gctattttca ccaaggcctt ccagagggat gtgttcatcc tactcagcaa gtttggcatc 2100
   tgtaaacgcc aggctcaggc ataccggggg cagagggttc ctccaaagaa cagcactgat 2160
   attcaggttc aaaaggttac ccacgagatg aggcagggtc tccacaacat ggaagatgtc 2220
   tatgaactga ttgaaaactc ccatctaacc ccaaagaagc aaggccaaat ctcagaagag 2280
   tatatgcaaa cggttttgta a 2301
<210> 6
   <211> 1995
   <212> DNA
   <213> Artificial
<220>
   <223> modified CNG alpha2
<400> 6
   atgatgaccg aaaaatccaa cggtgtgaag agctctccag ctaataacca taaccatcat    60
   cctcctcctt ctatcaaggc caatggcaaa gatgaccaca gggcaggtag cagaccacag   120
   tctgtggcag ctgatgatga cacttcctca gaactgcaaa ggttggcaga gatggatact   180
   ccacgcaggg gaaggggtgg cttccgaagg attgtccgcc tggtggggat catcagagac   240
   tgggccaaca aaaatttccg tgaggaggaa ccaagacctg actccttcct agagcgtttc   300
   cgtgggcctg agctccagac tgtgacaacc catcaggggg atggcaaagg cgacaaggac   360
   ggcgagggaa agggcaccaa aaagaaattt gaactgtttg ttttggaccc agctggagac   420
   tggtattacc gttggttgtt tgtcattgcc atgcctgttc tttacaactg gtgtctgttg   480
   gtagccagag cctgcttcag tgatctacag agaaactatt ttgtggtatg gctggtgctg   540
   gattacttct cagacactgt ttatattgca gacctcatca ttcggctgcg cacaggcttc 600
   ctagaacaag ggctcctggt caaagacccc aagaaattgc gagacaacta tatccacact 660
   ttgcagttca aattggatgt ggcttctatc atccccactg acctcattta ttttgctgtg 720
   ggtatccaca gccctgaggt acgctttaat cgtctgttac actttgcccg tatgtttgag 780
   ttctttgacc gcactgagac acgtacaagc taccccaaca tcttccgaat cagcaacctg 840
   gtcctctaca tcttggtcat catccactgg aatgcttgta tttactatgc tatctctaag 900
   tccattggct ttggggttga cacctgggtt taccccaaca ttactgaccc tgaatatggc 960
   tacctggcta gggagtacat ttactgcctt tactggtcca cactgaccct caccaccatt 1020
   ggagagacac caccccctgt aaaggatgag gagtacctat ttgtcatctt tgacttcctg 1080
   attggtgtcc tcatctttgc cactattgtg ggaaatgtgg gctccatgat ctccaacatg 1140
   aatgccacac gagcagagtt ccaggccaag attgatgctg tcaaacacta catgcagttc 1200
   cgaaaggtca gtaaagacat ggaagccaag gtcatcaaat ggtttgacta cttgtggacc 1260
   aataagaaga cagtagatga acgagaagtc ctaaagaacc tgcctgcaaa gcttagggca 1320
   gagatagcca ttaatgttca tttgtccact ctgaagaaag tgcgcatatt ccaggattgg 1380
   gaagctggcc tgctggtgga actggtactg aagcttcgtc ctcaggtctt tagtcctgga 1440
   gattatattt gccgtaaggg ggacattggc aaggaaatgt acatcatcaa ggagggcaaa 1500
   ttggcagtgg tagctgatga tggtgtgact cagtatgcct tgctgtcagc tgggagctgc 1560
   tttggtgaga ttagtatcct taacattaag ggtagcaaaa tgggcaatcg gcgcactgcc 1620
   aatatccgta gtctgggcta ctcagatctc ttctgcttgt ccaaggatga tcttatggaa 1680
   gctgtgactg agtatcctga tgccaagaaa gtcctggaag aacggggtag ggagatcctg 1740
   atgaagatgg gtctactgga tgagaatgaa gtggcagcta gtatggaggt agatgttcag 1800
   gagaagctgg agcagctgga gacaaacatg gagaccttgt acactcgttt tgcccgcctg 1860
   ctggctgagt acactggagc ccagcagaag ctcaaacagc gcatcacagt gctggagacc 1920
   aagatgaaac agaaccatga ggatgattac ctatcagatg ggataaacac ccctgagcca 1980
   gctgttgctg aatag 1995
<210> 7
   <211> 687
   <212> DNA
   <213> Artificial
<220>
   <223> modified aequorin
<400> 7
   atgagcgtgc tgacccccct gctgctgcgc ggcctgaccg gcagcgcccg ccgcctgccc      60
   gtgccccgcg ccaagatcca cagcctgccc cccgagggca agctgggcat catgaaggtg     120
   aagctgacca gcgacttcga caaccccaag tggatcggcc gccacaagca catgttcaac     180
   ttcctggacg tgaaccacaa cggccgcatc agcctggacg agatggtgta caaggccagc     240
   gacatcgtga tcaacaacct gggcgccacc cccgagcagg ccaagcgcca caaggacgcc     300
   gtggaggcct tcttcggcgg cgccggcatg aagtacggcg tggagaccga gtggcccgag     360
   tacatcgagg gctggaagcg cctggccacc gaggagctgg agcgctacag caagaaccag     420
   atcaccctga tccgcctgtg gggcgacgcc ctgttcgaca tcatcgacaa ggaccagaac     480
   ggcgccatca ccctggacga gtggaaggcc tacaccaaga gcgccggcat catccagagc     540
   agcgaggact gcgaggagac cttccgcgtg tgcgacatcg acgagagcgg ccagctggac     600
   gtggacgaga tgacccgcca gcacctgggc ttctggtaca ccatggaccc cgcctgcgag     660
   aagctgtacg gcggcgccgt gccctaa                                          687